# EUROPEAN PATENT SPECIFICATION

(11) **EP 2 938 258 B1**
(45) Date of publication and mention of the grant of the patent: **30.05.2018**
(21) Application number: 13821988.6
(22) Date of filing: 27.12.2013
(51) Int. Cl.: A61B 5/145, A61B 5/00, A61B 5/1459, G01N 33/543

(54) **APPARATUS FOR FACILITATING CELL GROWTH IN AN IMPLANTABLE SENSOR**
VORRICHTUNG ZUR ERLEICHTERUNG DES ZELLWACHSTUMS IN EINEM IMPLANTIERBAREN SENSOR
APPAREIL POUR FACILITER LA CROISSANCE CELLULAIRE DANS UN DÉTECTEUR POUVANT ÊTRE IMPLANTÉ

(30) Priority: 28.12.2012 US 201261746691 P
(43) Date of publication of application: 04.11.2015
(73) Proprietor: Glusense, Ltd., 76702 Rehovot (IL)
(72) Inventor: BRILL, Boaz, Rehovot (IL); WEISMAN, Itamar, 99793 Yad Rambam (IL); GLADNIKOFF, Micha, Tel Aviv (IL); ROMANO, Jacob, Tel Aviv (IL); GIL, Tamir, 38930 Kibbutz Givat Haim Meuchad (IL)
(74) Representative: Evens, Paul Jonathan
(86) International application number: PCT/IB2013/061368
(87) International publication number: WO 2014/102743

(56) References cited:
- US-A1- 2004 133 188
- US-A1- 2008 166 329
- US-A1- 2008 166 329
- US-A1- 2010 202 966
- US-A1- 2010 202 966
- US-A1- 2012 059 232
- US-A1- 2012 113 997

## Description

### CROSS-REFERENCE TO RELATED APPLICATIONS

The present application claims priority from US Provisional Application 61/746,691, filed December 28, 2012, which is assigned to the assignee of the present application.

### FIELD OF THE INVENTION

Some applications of the present invention relate generally to implantable sensors for detecting an analyte in a body and specifically to methods and apparatus for providing nutrients to cells in an implantable medical device.

### BACKGROUND OF THE INVENTION

The monitoring of various medical conditions often requires measuring the levels of various components within the blood. In order to avoid invasive repeated blood drawing, implantable sensors aimed at detecting various components of blood in the body have been developed. More specifically, in the field of endocrinology, in order to avoid repeated "finger-sticks" for drawing blood to assess the levels of glucose in the blood in patients with diabetes mellitus, implantable glucose sensors have been discussed.

One method for sensing the concentration of an analyte such as glucose relies on Fluorescence Resonance Energy Transfer (FRET). FRET involves the transfer of non-photonic energy from an excited fluorophore (the donor) to another fluorophore (the acceptor) when the donor and acceptor molecules are in close proximity to each other. FRET enables the determination of the relative proximity of the molecules for investigating, for example, the concentration of an analyte such as glucose.

PCT Patent Application Publication WO 2006/006166 to Gross et al., describes a protein which includes a glucose binding site, cyan fluorescent protein (CFP), and yellow fluorescent protein (YFP). The protein is configured such that binding of glucose to the glucose binding site causes a reduction in a distance between the CFP and the YFP. Apparatus is described for detecting a concentration of a substance in a subject, the apparatus comprising a housing adapted to be implanted in the subject. The housing comprises a fluorescence resonance energy transfer (FRET) measurement device and cells genetically engineered to produce, in situ, a FRET protein having a FRET complex comprising a fluorescent protein donor, a fluorescent protein acceptor, and a binding site for the substance. US 2008/166329 A1 discloses an implantable encapsulation device comprising cell monolayers attached to a three-dimensional scaffold.

### SUMMARY OF THE INVENTION

The subject-matter of the present invention is defined by the scope of the claims. One of the challenges in the design of a cell-based implantable device is the maintenance of a significant population of cells over the long term, e.g., over a year or longer. In accordance with some applications of the present invention, techniques are provided for maintaining a desired cell population size over time, including both:
- restraining cell population growth, i.e., cell proliferation, in order to avoid over-population that leads to a shortage of nutrients in cells farther from the edge of the device, which would create a necrotic core of cells that eventually intoxicates the entire cell population; and
- allowing limited cell proliferation to replace cells that die over time, in order to prevent dwindling of the cell population in the device, which would eventually render the device dysfunctional.

In order to balance the above-mentioned conflicting goals and preserve a generally constant cell population over a long period of time, e.g., at least one year, a three-layer cell encapsulation structure is provided, which comprises a substantially non-degradable three-dimensional scaffold having surfaces to which cells are attached, and a hydrogel, which is applied to the cells.

The scaffold, cells, and hydrogel are arranged such that the cells are sandwiched in spaces between the hydrogel and the surfaces of the scaffold. The cells are arranged in monolayers on at least 50% of an aggregate surface area of the surfaces of the scaffold. This arrangement allows mobility and proliferation of the cells in the spaces between the hydrogel and the surfaces of the scaffold, and prevents the mobility and the proliferation of the cells to locations outside of the spaces between the hydrogel and the surfaces of the scaffold. Cells within the spaces between the hydrogel and the surfaces of the scaffold that die leave a space upon disintegration. The structure provided by the surface of the scaffold on one side and the hydrogel on the other side maintain the patency of this space until one or more neighboring cells proliferate into the space.

Thus, in any local microscopic environment the encapsulation structure comprises a three-layer stack of (a) the surface of the solid scaffold, (b) the cells, and (c) the hydrogel, in this order. The cells at any location are thus generally limited to a monolayer, allowing free mobility and proliferation of the cells within the narrow space between the scaffold and the hydrogel, but preventing any proliferation into the rest of the volume and creation of three-dimensional cell structures.

The scaffold provides a three-dimensional structure with a high aggregate surface area, and high surface-to-volume ratio, which makes efficient use of the three-dimensional volume of the chamber. The surfaces of the scaffold, although often not flat, serve effectively as a two-dimensional substrate for seeding, growth, and attachment of the cells. If the hydrogel were not provided over the monolayer of the cells, the cells typically grow in three dimensions, away from the surfaces to which they are attached. Such three-dimensional growth would generally result in undesirable over-population, as described above. In addition, for many cell types, cell viability and protein expression, including expression of the sensor protein, are significantly enhanced when cells are attached and spread. Thus cells in this configuration will survive longer and function better than suspended cells, e.g., cells suspended in a hydrogel scaffold.

For some applications, the scaffold comprises microcarrier beads, fibers, a rigid structure, or a sponge structure having a plurality of interconnected internal pores.

The encapsulation structure may combine at least three benefits: (a) good cell attachment, leading to better cell viability and expression, lacking in simpler systems that for example use hydrogel as a scaffold, (b) prevention of over-population which often leads to a necrotic core, because of a limited number of cells and open diffusion channels to the cells via the hydrogel, and (c) enablement of cell mobility and proliferation within a two-dimensional culture, thereby enabling long-term steady state population.

Some applications of the present invention provide a multi-layer immunoisolation system. The viability of cells within a cell-based device strongly depends on an ample supply of oxygen. Generally, the foreign body response following device implantation creates dense fibrotic tissue that encapsulates the device, substantially reducing oxygen diffusion to the device from the blood circulation. Therefore, the viability of cells inside a cell-based device is enhanced by substantial vascularization of the tissue as close as possible to the implanted device, which increases oxygen levels at the device surface. More specifically, for a glucose measurement device, the creation of a dense fibrotic tissue is a potential diffusion barrier for glucose, leading to a time delay between glucose levels in the tissue and glucose levels measured by the device. Such dense fibrotic tissue should thus be avoided in order to maintain the accuracy of the glucose measurement.

The multi-layer immunoisolation system is configured to enhance long-term function of an implanted cell-based device. The multi-layer immunoisolation system comprises at least the following three layers: (a) a lower (inner) membrane layer, which is disposed at an external surface of the device, (b) an upper (outer) neovascularization layer, and (c) a middle protective layer, disposed between the lower membrane layer and the upper neovascularization layer. The multi-layer immunoisolation system comprises a biodegradable scaffold. Before biodegrading, the biodegradable scaffold spans both the upper neovascularization layer and the middle protective layer, such that the upper neovascularization layer comprises a first upper portion of the biodegradable scaffold, and the middle protective layer comprises a second lower portion of the biodegradable scaffold. In addition, the middle protective layer further comprises a non-biodegradable hydrogel that impregnates the second lower portion of the biodegradable scaffold. The upper neovascularization layer, which comprises the first upper portion of the biodegradable scaffold, is not impregnated with the hydrogel.

The biodegradable scaffold serves at least two functions: (a) during implantation of the device, the biodegradable scaffold protects the soft hydrogel of the middle protective layer from strong shear forces which might otherwise pull off the soft hydrogel; and (b) after implantation of the device, the biodegradable scaffold promotes vascularization of the tissue that grows into the upper neovascularization layer, until the biodegradable scaffold eventually degrades and is totally absorbed.

Upon biodegradation of the biodegradable scaffold, the middle protective layer (now comprising primarily the hydrogel) remains attached to the lower membrane layer. The middle protective layer typically serves to (a) prevent attachment of proteins to the lower membrane layer, thereby minimizing the creation of a fibrotic tissue, and/or (b) repel large proteins, thereby minimizing the fouling of the lower membrane layer. The high water content of the hydrogel of the middle protective layer prevents the attachment of various proteins, so that immune system cells are less likely to attach to the tissue-hydrogel interface, thereby minimizing the overall immune response. As a result of this triple-layer protection, the tissue surrounding the device is characterized by high vascularization and minimal fibrosis.

Some applications of the present invention provide another multi-layer immunoisolation system, which comprises at least the following three layers: (a) a lower (inner) membrane layer, which is disposed at an external surface of the device, (b) an upper (outer) protective layer, and (c) a middle attachment layer, which is disposed between the lower membrane layer and the upper protective layer, and which tightly fixes the upper protective layer to the lower membrane layer. The middle attachment layer comprises a non-biodegradable scaffold, which is tightly fixed to the lower membrane layer, such as by being deposited directly on the membrane using electro spinning.

The multi-layer immunoisolation system comprises a non-biodegradable hydrogel, which spans both the upper protective layer and the middle attachment layer. In other words, the middle attachment layer comprises a first portion of the hydrogel, and the upper protective layer comprises a second portion of the hydrogel. The hydrogel is impregnated in the scaffold of the middle attachment layer, and extends above the scaffold, i.e., in a direction away from the lower membrane, so as to provide the upper protective layer. The upper protective layer does not comprise the scaffold. As a result, the scaffold is not exposed to tissue, thereby reducing the likelihood that the multi-layer immunoisolation system generates an immune response.

The middle attachment layer holds the hydrogel of the upper protective layer in place on the lower membrane layer. The upper protective layer has a smooth upper (outer) surface, which results in low biofouling of the lower membrane layer, allowing the membrane to efficiently diffuse nutrients into the device even after a long implantation period. In addition, the upper protective layer protects the device by presenting a highly biocompatible surface to the tissue.

In some applications of the present invention, a fully-implantable or partially-implantable sensor device comprises apparatus for facilitating cell growth. For some applications, the apparatus comprises a chamber and a membrane that surrounds the chamber at least in part and is permeable to nutrients. ("Nutrients," in the context of the specification and in the claims, includes oxygen, glucose, and other molecules important for cell survival.) Typically, a scaffold comprising a hydrogel or other suitable material is disposed within the chamber, and a plurality of cells is disposed therein. Additionally, at least one nutrient supply compartment is typically disposed within the chamber, and interspersed with the scaffold such that at least 80% of the cells within the cell-growth medium are disposed within 100 um (microns) of the nutrient supply compartment. In this manner, the nutrient supply compartment is positioned within the chamber such that a diffusion path for nutrients is provided, by the nutrient supply compartment, between the membrane and the at least 80% of the cells.

In the context of the present application and in the claims, a membrane which is described as "surrounding" an element is to be understood as surrounding the element at least in part. Thus, for example, a membrane that surrounds a chamber may entirely surround the chamber, or may surround the chamber in part (while another portion of the chamber may be covered with something other than the membrane).

In some applications of the present invention, the apparatus comprises a chamber having isolated cells disposed (e.g., encapsulated) therein, and a membrane structure that surrounds the chamber at least in part. The membrane structure in a first state thereof has a first molecular weight cut off (MWCO), and transitions to a second state thereof, in which the membrane structure has a second MWCO, the second MWCO being higher than the first MWCO. One of the goals of the apparatus is to maintain a constant flow of nutrients into the chamber by increasing membrane permeability, thus reducing the adverse effect on nutrient flow due to membrane fouling (which otherwise may limit nutrient flow to the cells). The membrane structure is generally impermeable to white blood cells, for many months or even the entire time that the apparatus is implanted in the subject. Permeability to large molecules such as transferrin or even IgG increases over time.

It is noted that even though this application is described hereinabove and below as having the second MWCO being higher than the first MWCO, the scope of the present invention includes applications in which the first MWCO is the same or even larger than the second MWCO. A benefit in such an application case is enhanced total membrane thickness, which reduces the effect of some of the immune system components, especially reactive oxygen species (ROS). Additionally, a soft biodegradable membrane as provided by some applications of the present invention may recruit a weaker immune response compared to a rigid surface.

In some applications of the present invention, the membrane structure comprises (a) a first layer comprising a biodegradable material, and (b) a second layer that is non-biodegradable. In some applications, the membrane structure comprises a non-biodegradable material impregnated with a biodegradable material. Over time, the biodegradable material biodegrades, thereby leaving spaces in the non-biodegradable material, thereby increasing the permeability of the membrane structure. As a result the membrane structure initially has a low MWCO, which is effective for blocking cytokines, while after the biodegradable material has degraded, the non-biodegradable material having the larger MWCO remains. Thus, even if there has been fouling of the membrane, nutrients can still pass through the membrane due to the higher permeability of the membrane caused by degradation of the biodegradable material.

In some applications of the present invention, the apparatus comprises an optically-transparent scaffold; an optical waveguide, coupled to the scaffold; a plurality of cells on the scaffold; and a membrane structure surrounding the scaffold. The transparency of the scaffold enables light to pass through the optical waveguide to the scaffold, and through the scaffold to where the sensor protein secreted from the cells or produced within the cells are disposed. Similarly, fluorescent light emitted by the sensor protein in response to the excitation light is transmitted through the transparent scaffold to the optical waveguide.

In some applications of the present invention, apparatus for detecting a concentration of an analyte in a subject comprises an optical waveguide having a proximal end and a distal end. A sensing unit is disposed at the distal end of the optical waveguide and detects the analyte (e.g., by the binding of the analyte to a protein). The sensing unit comprises a first chamber. A second chamber is disposed around at least a distal end portion of the first chamber. Live cells that are genetically engineered to produce, in the body of the subject, a sensor protein having a binding site for the analyte, are disposed (e.g., encapsulated) within either the first chamber or the second chamber.

In some applications of the present invention, apparatus for detecting a concentration of an analyte in a subject comprises an optical waveguide having a first, distal, end and a second, proximal, end. A sensing unit for detecting analyte is disposed at the first end of the optical waveguide. The sensing unit comprises at least an inner axial portion without cells, disposed adjacent to the first end of the optical waveguide. A second chamber is adjacent to the inner axial portion, and is coaxial with the optical waveguide and the inner axial portion. Live cells that are genetically engineered to produce, in the subject's body, a sensor protein having a binding site for the analyte, are disposed in the second chamber and secrete a sensor protein. In this configuration, a relatively large surface area is provided for allowing transfer of analyte and nutrients between the subject's body and the second chamber.

In some applications of the present invention, the apparatus for detecting a concentration of an analyte in a subject comprises an optical waveguide; a chamber surrounding a distal portion of the optical waveguide, the distal portion of the optical waveguide extending along at least 75% of a length of the chamber; and live cells that are genetically engineered to produce, in a body of the subject, a sensor protein having a binding site for the analyte. The live cells are disposed (e.g., encapsulated) within the chamber.

In some applications of the present invention the apparatus for detecting a concentration of an analyte in a subject comprises an optical waveguide that transmits excitation light, and a chamber comprising (i.e., containing) live cells that are genetically engineered to produce, in a body of the subject, a fluorescent sensor protein having a binding site for the analyte. The fluorescent sensor protein is configured to emit fluorescent light in response to the excitation light. The chamber is disposed coaxially with respect to the optical waveguide. A lens is disposed between the optical waveguide and the chamber, the lens configured to focus light from the optical waveguide to the chamber and from the chamber to the optical waveguide. A first mirror is coupled to the chamber, and is disposed between a proximal end of the chamber and the lens. The first mirror reflects the excitation light within the chamber and transmits the fluorescent light from within the chamber toward the lens and the optical waveguide. The first mirror is shaped to define a pinhole that allows passage of the excitation light from the lens into the chamber. A second mirror is coupled to the chamber and disposed at a distal end of the chamber.

Applications of the present invention also include a method for facilitating the measurement of a concentration of an analyte in a body of a subject, from a subcutaneous location of the subject. This is accomplished by measuring a temperature of the subcutaneous location in conjunction with the facilitating of the measuring of the concentration of the analyte; and calibrating the measurement of the concentration of the analyte in response to the measured temperature.

There is therefore provided, in accordance with an application of the present invention, apparatus for detecting a concentration of an analyte in a subject, the apparatus configured to be implanted in a body of the subject and including:
an optical waveguide having a proximal end and a distal end;
a sensing unit disposed at the distal end of the optical waveguide and configured to detect the analyte, the sensing unit including:
   at least a first chamber;
   at least a second chamber disposed around the first chamber at least at a proximal end portion of the first chamber; and
   live cells that are genetically engineered to produce, in a body of the subject, a sensor protein having a binding site for the analyte, the live cells being disposed within at least one chamber selected from the group consisting of: the first chamber and the second chamber.

For some applications, the analyte is glucose.

For some applications, the second chamber completely surrounds the first chamber.

For some applications, the optical waveguide includes an optical fiber. For some applications, the optical waveguide includes a planar optical waveguide.

For some applications, the live cells are disposed within the first chamber. For some applications, a first distal longitudinal segment of the second chamber is disposed around the first chamber at least at the proximal end portion of the first chamber, and a second proximal longitudinal segment of the second chamber does not surround the first chamber. For some applications, at least 60% of a volume of the second chamber is disposed along the second proximal longitudinal segment. For some applications, the first longitudinal segment of the second chamber completely surrounds the first chamber. For some applications, a diameter of the optical waveguide is equal to a diameter of the second chamber.

For some applications, the optical waveguide has a diameter that is equal to a diameter of the first chamber.

For some applications, the apparatus further includes a first semi-permeable membrane between the first and second chambers, the live cells are genetically engineered to secrete the sensor protein, and the semi-permeable membrane is configured to facilitate passage of the sensor protein from the first chamber to the second chamber and restrict passage of the live cells therethrough. For some applications, the second chamber completely surrounds the first chamber. For some applications, the optical waveguide has a diameter that is equal to an outer diameter of the second chamber. For some applications, the apparatus further includes a second semi-permeable membrane surrounding the second chamber, the second semi-permeable membrane being configured to facilitate passage of nutrients into the second chamber and restrict cell passage therethrough.

For some applications, the first chamber has a proximal portion and a distal portion, and the proximal portion has a proximal-portion diameter that is smaller than a distal-portion diameter of the distal portion. For some applications, a diameter of the optical waveguide is equal to the distal-portion diameter.

For some applications, the second chamber surrounds the proximal portion of the first chamber, and the second chamber facilitates passage of nutrients to the live cells in the first chamber from fluid of the subject. For some applications, the apparatus further includes a semi-permeable membrane between the first and second chambers, the live cells are genetically engineered to secrete the sensor protein, and the semi-permeable membrane is configured to facilitate passage of the sensor protein from the first chamber to the second chamber. For some applications, the apparatus further includes a semi-permeable membrane surrounding at least one chamber selected from the group consisting of: the first and second chambers, the second semi-permeable membrane being configured to facilitate passage of nutrients into the selected chamber and restrict cell passage therethrough. For some applications, the semi-permeable membrane is configured to restrict cell passage into the second chamber. For some applications, the semi-permeable membrane is configured to restrict passage of the cells into the first chamber.

For some applications, the live cells are disposed within the second chamber. For some applications, a first proximal longitudinal segment of the second chamber completely surrounds the first chamber, and a second distal longitudinal segment of the second chamber does not surround the first chamber. For some applications, at least 60% of a volume of the second chamber is disposed along the second distal longitudinal segment. For some applications, a diameter of the optical waveguide is equal to a diameter of the first chamber.

For some applications, the apparatus further includes a first semi-permeable membrane between the first and second chambers, the live cells are genetically engineered to secrete the sensor protein, and the semi-permeable membrane is configured to facilitate passage of the sensor protein from the first chamber to the second chamber. For some applications, the optical waveguide has a diameter that is equal to a diameter of the first chamber.

For some applications, the optical waveguide has a diameter that is equal to a diameter of the second chamber.

For some applications, the first chamber includes optically-transparent material configured to transmit light through the first chamber. For some applications, the apparatus further includes a mirror disposed at a distal end of the first chamber and configured to reflect transmitted light through the first chamber.

For some applications, the first chamber includes optically-transparent material configured to transmit light through the first chamber. For some applications, the apparatus further includes a mirror disposed at a distal end of the first chamber and configured to reflect transmitted light through the first chamber.

There is further provided, in accordance with an application of the present invention, apparatus containing cells for implantation into a human subject, the apparatus including:
a substantially non-degradable three-dimensional scaffold having surfaces to which the cells are attached; and
a hydrogel, which is attached to the cells,
wherein the scaffold, the cells, and the hydrogel are arranged such that the cells are sandwiched in spaces between the hydrogel and the surfaces of the scaffold, and
wherein the cells are arranged in monolayers on at least 50% of an aggregate surface area of the surfaces of the scaffold, thereby allowing mobility and proliferation of the cells in the spaces between the hydrogel and the surfaces of the scaffold, and preventing the mobility and the proliferation of the cells to locations outside of the spaces between the hydrogel and the surfaces of the scaffold.

For some applications, the cells are arranged in the monolayers on at least 70% of the aggregate surface area of the surfaces of the scaffold, such as on at least 90% of the aggregate surface area of the surfaces of the scaffold.

For some applications, the apparatus further includes a chamber, in which the scaffold, the cells, and the hydrogel are contained. For some applications, the apparatus further includes an external membrane, which surrounds at least a portion of the chamber.

For some applications, the cells are differentiated cells, such as terminally-differentiated cells, which are attached to the surfaces of the scaffold. Alternatively, for some applications, the cells are stem cells, which are attached to the surfaces of the scaffold.

For some applications, the cells are genetically engineered to produce a fluorescent sensor protein having a binding site for an analyte, the fluorescent sensor protein being configured to emit fluorescent light in response to excitation light. For some applications, the analyte is glucose.

For any of the applications described above, the scaffold may include microcarrier beads.

For any of the applications described above, the scaffold may include fibers.

For any of the applications described above, the scaffold may include a sponge structure having a plurality of interconnected internal pores.

For any of the applications described above, the scaffold may be rigid. For some applications, the rigid scaffold is shaped so as to define a plurality of wells.

There is still further provided, in accordance with an application of the present invention, a method for manufacturing a cell encapsulation structure, including:
providing a substantially non-degradable three-dimensional scaffold having surfaces suitable for cell attachment and growth;
seeding cells on the surfaces and allowing cell proliferation to reach at least 70% confluence; and
before the cells form three-dimensional structures on 50% of an aggregate surface area of the surfaces, filling, with a hydrogel, a volume of the cell encapsulation structure which is not already occupied by the cells or the scaffold, thereby preventing additional cell proliferation into the volume of the cell encapsulation structure which is not already occupied by the cells or the scaffold.

There is additionally provided, in accordance with an application of the present invention, apparatus including a multi-layer immunoisolation system for application to an implantable cell-based device, the multi-layer immunoisolation system including:
a lower membrane layer, which is disposed at an external surface of the device;
an upper neovascularization layer, which includes a first upper portion of the biodegradable scaffold; and
a middle protective layer, which (a) is disposed between the lower membrane layer and the upper neovascularization layer, and (b) includes:
   a second lower portion of the biodegradable scaffold, which is fixed to the lower membrane layer; and
   a non-biodegradable hydrogel that impregnates the second lower portion of the biodegradable scaffold,
wherein the upper neovascularization layer is not impregnated with the hydrogel.

For some applications, the lower membrane layer has a molecular weight cutoff (MWCO) of between 5 and 50 KDa.

For some applications, the biodegradable scaffold has a thickness of between 100 and 300 microns.

For some applications, the biodegradable scaffold includes a polymer.

For some applications, the lower membrane layer includes a material selected from the group consisting of: polysulfone (PS), polyethersulfone (PES), modified polyethersulfone (mPES), and polytetrafluoroethylene (PTFE).

For any of the applications described above, the biodegradable scaffold may be electrospun onto the lower membrane layer.

There is yet additionally provided, in accordance with an application of the present invention, apparatus including a multi-layer immunoisolation system for application to an implantable cell-based device, the multi-layer immunoisolation system including:
a non-biodegradable hydrogel;
a lower membrane layer, which is disposed at an external surface of the device;
an upper protective layer, which includes a first portion of the hydrogel; and
a middle attachment layer, which (a) is disposed between the lower membrane layer and the upper protective layer, and (b) includes:
   a non-biodegradable scaffold, which is fixed to the lower membrane layer; and
   a second portion of the hydrogel, which is impregnated in the scaffold, wherein the upper protective layer does not include the scaffold.

For some applications, the lower membrane layer has a molecular weight cutoff (MWCO) of between 5 and 50 KDa.

For some applications, the lower membrane layer includes a material selected from the group consisting of: polysulfone (PS), polyethersulfone (PES), modified polyethersulfone (mPES), and polytetrafluoroethylene (PTFE).

For some applications, the non-biodegradable scaffold includes a polymer.

For some applications, the middle attachment layer has a thickness of between 50 and 150 microns.

For some applications, the upper protective attachment layer has a thickness of between 50 and 200 microns.

For any of the applications described above, the non-biodegradable scaffold may be electrospun onto the lower membrane layer.

There is also provided in accordance with an inventive concept 1, apparatus for facilitating cell growth, the apparatus configured to be implanted in a body of a subject and comprising:
a chamber;
a membrane that surrounds the chamber at least in part and is permeable to nutrients;
a scaffold within the chamber, the scaffold having at least 1000 cells coupled thereto; and
at least one nutrient supply compartment within the chamber, interspersed with the scaffold such that at least 80% of the cells coupled to the scaffold are disposed within 100 microns of the nutrient supply compartment, the nutrient supply compartment being positioned within the chamber such that a diffusion path for nutrients is provided, by the nutrient supply compartment, between the membrane and the at least 80% of the cells..

Inventive concept 2. The apparatus according to inventive concept 1, wherein a volume of the nutrient supply compartment is 25%-75% of a volume of the chamber.

Inventive concept 3. The apparatus according to inventive concept 1, wherein the scaffold has at least 2000 cells coupled thereto.

Inventive concept 4. The apparatus according to inventive concept 1, wherein the scaffold has fewer than 20,000 cells coupled thereto.

Inventive concept 5. The apparatus according to inventive concept 4, wherein the scaffold has fewer than 10,000 cells coupled thereto.

Inventive concept 6. The apparatus according to inventive concept 1, wherein at least 80% of the cells coupled to the scaffold are disposed within 50 microns of the nutrient supply compartment.

Inventive concept 7. The apparatus according to claim 1, wherein the scaffold comprises a hydrogel.

Inventive concept 8. The apparatus according to any one of inventive concepts 1-7, further comprising a nutrient permeable medium that is disposed within the nutrient supply compartment and that is not conducive to cell growth.

Inventive concept 9. The apparatus according to inventive concept 8, wherein the nutrient permeable medium comprises a material selected from the group consisting of: silicone rubber, fused glass powder, sintered glass powder, a hydrogel, and alginate.

Inventive concept 10. The apparatus according to inventive concept 8, wherein the nutrient permeable medium is shaped to define one or more spheres.

Inventive concept 11. The apparatus according to inventive concept 10, wherein the one or more spheres comprises 100-1000 spheres.

Inventive concept 12. The apparatus according to inventive concept 10, wherein a volume of the chamber is at least 20 times a volume of at least one of the spheres.

Inventive concept 13. The apparatus according to inventive concept 12, wherein the volume of the chamber is at least 100 times the volume of the at least one of the spheres.

Inventive concept 14. The apparatus according to inventive concept 13, wherein the volume of the chamber is 200-1000 times the volume of the at least one of the spheres.

Inventive concept 15. The apparatus according to inventive concept 10, wherein the spheres are disposed in the chamber in an efficient packing configuration.

There is further provided in accordance with an inventive concept 16, apparatus for facilitating cell growth, the apparatus configured to be implanted in a body of a subject and comprising:
a chamber having cells disposed therein; and
a membrane structure that surrounds the chamber at least in part, which membrane structure in a first state thereof has a first molecular weight cut off (MWCO), and which is configured to transition to a second state thereof, in which the membrane structure has a second MWCO, the second MWCO being higher than the first MWCO.

Inventive concept 17. The apparatus according to inventive concept 16, wherein the membrane structure is permeable to nutrients.

Inventive concept 18. The apparatus according to inventive concept 16, wherein the second molecular weight cutoff (MWCO) is at least three times higher than the first MWCO.

Inventive concept 19. The apparatus according to inventive concept 16, wherein the second MWCO is greater than 150 kilodaltons.

Inventive concept 20. The apparatus according to inventive concept 16, wherein the membrane structure in the first state is not permeable to IgG.

Inventive concept 21. The apparatus according to inventive concept 20, wherein the membrane structure in the second state is permeable to IgG.

Inventive concept 22. The apparatus according to inventive concept 16, wherein the membrane structure in the first state is permeable to glucose and not permeable to IgG.

Inventive concept 23. The apparatus according to inventive concept 22, wherein the membrane structure in the second state is permeable to glucose and permeable to IgG.

Inventive concept 24. The apparatus according to inventive concept 16, wherein the membrane structure in the first state is not permeable to transferrin.

Inventive concept 25. The apparatus according to inventive concept 24, wherein the membrane structure in the second state is permeable to transferrin.

Inventive concept 26. The apparatus according to inventive concept 16, wherein the membrane structure in the first and second states is not permeable to white blood cells. Inventive concept 27. The apparatus according to inventive concept 16, wherein the first MWCO is less than 150 kilodaltons.

Inventive concept 28. The apparatus according to inventive concept 27, wherein the first MWCO is less than 100 kilodaltons.

Inventive concept 29. The apparatus according to inventive concept 28, wherein the first MWCO is less than 50 kilodaltons.

Inventive concept 30. The apparatus according to inventive concept 16, wherein the second MWCO is greater than two times the first MWCO, and wherein the first MWCO is less than 100 kilodaltons.

Inventive concept 31. The apparatus according to any one of inventive concepts 16-30, wherein the membrane structure comprises:
a first material that is biodegradable and has the first MWCO; and
a second material, which is non-biodegradable and has the second MWCO.

Inventive concept 32. The apparatus according to inventive concept 31, wherein the first material has a thickness of 50-500 microns.

Inventive concept 33. The apparatus according to inventive concept 31, wherein the second material is impregnated with the first material.

Inventive concept 34. The apparatus according to inventive concept 31, wherein the first material is configured to biodegrade in the presence of body fluids within a period of two weeks to six months.

Inventive concept 35. The apparatus according to inventive concept 31, wherein the second material is permeable to molecules that are 80-300 kilodaltons.

Inventive concept 36. The apparatus according to inventive concept 31, wherein the membrane structure comprises:
a first layer, comprising the first material; and
a second layer, comprising the second material.

Inventive concept 37. The apparatus according to inventive concept 36, wherein the second layer is disposed between the cells and the first layer.

Inventive concept 38. The apparatus according to inventive concept 36, wherein the non-biodegradable material comprises a material selected from the group consisting of: polysulfone (PSU), polytetrafluoroethylene (pTFE), and polyethersulfone (PES).

Inventive concept 39. The apparatus according to inventive concept 36, wherein the first layer has a thickness of 50-500 microns.

Inventive concept 40. The apparatus according to inventive concept 36, wherein the second layer has a thickness of 50-250 microns.

Inventive concept 41. The apparatus according to inventive concept 36, wherein the biodegradable material comprises a hydrogel.

Inventive concept 42. The apparatus according to any one of inventive concepts 16-30, further comprising:
a scaffold to which the cells are attached; and
a scaffold to which the cells are attached; and
a nutrient supply compartment disposed at least partially between the scaffold and the membrane structure, the nutrient supply compartment being permeable to nutrients and configured to inhibit growth of the cells into the nutrient supply compartment.

Inventive concept 43. The apparatus according to inventive concept 42, further comprising an optical waveguide, wherein the scaffold is: (a) coupled to the optical waveguide, and (b) configured to facilitate illumination of the chamber by the optical waveguide.

Inventive concept 44. The apparatus according to inventive concept 43, wherein the optical waveguide comprises an optical fiber.

Inventive concept 45. The apparatus according to inventive concept 43, wherein the optical waveguide comprises a planar optical waveguide.

Inventive concept 46. The apparatus according to inventive concept 42, wherein the scaffold mechanically supports the membrane structure.

Inventive concept 47. The apparatus according to inventive concept 42, wherein the scaffold is optically transparent.

Inventive concept 48. The apparatus according to inventive concept 42, wherein the scaffold comprises a material selected from the group consisting of: a hydrogel, molded plastic and polystyrene.

Inventive concept 49. The apparatus according to inventive concept 42, wherein the nutrient supply compartment is optically transparent.

There is still further provided in accordance with an inventive concept 50, apparatus for facilitating cell growth, the apparatus configured to be implanted in a body of a subject and comprising:
an optically-transparent rigid scaffold;
an optical waveguide, coupled to the scaffold;
a plurality of cells disposed on the scaffold; and
a membrane structure at least partially surrounding the scaffold.

Inventive concept 51. The apparatus according to inventive concept 50, wherein the optical waveguide comprises an optical fiber.

Inventive concept 52. The apparatus according to inventive concept 50, wherein the optical waveguide comprises a planar optical waveguide.

Inventive concept 53. The apparatus according to inventive concept 50, wherein the scaffold is shaped to define one or more wells in which cell-growth medium is disposed, and on which the cells are disposed.

Inventive concept 54. The apparatus according to inventive concept 53, wherein a total surface area of the scaffold upon which the cells are disposed is at least 60% of a total surface area of the scaffold which is illuminated when light passes through the optical waveguide.

Inventive concept 55. The apparatus according to inventive concept 50, wherein the cells form a monolayer on the scaffold.

Inventive concept 56. The apparatus according to inventive concept 50, wherein a length of the scaffold is 2-4 mm.

Inventive concept 57. The apparatus according to inventive concept 50, wherein a volume of the scaffold is 0.5-2 microliter.

Inventive concept 58. The apparatus according to inventive concept 50, wherein a total surface area of the scaffold upon which the cells are disposed is 2.5-3.5 mm^2.

Inventive concept 59. The apparatus according to inventive concept 50, wherein (a) within an exit cone of twenty-two degrees from a tip of the optical waveguide, and (b) within a distance from the tip, the distance being four times a diameter of the waveguide, (c) there is a scaffold surface area for cell growth that is at least four times the surface area of a distal tip of the waveguide.

Inventive concept 60. The apparatus according to any one of inventive concepts 50-59, wherein the scaffold has a plurality of surfaces perpendicular to the optical waveguide.

Inventive concept 61. The apparatus according to inventive concept 60, wherein the plurality of cells do not secrete a sensor protein.

Inventive concept 62. The apparatus according to inventive concept 60, wherein the plurality of cells secrete a sensor protein.

Inventive concept 63. The apparatus according to any one of inventive concepts 50-59, further comprising a mirror coupled to the scaffold and configured to reflect light, transmitted from the optical waveguide, back to the optical waveguide.

Inventive concept 64. The apparatus according to inventive concept 63, wherein the mirror is not flat.

Inventive concept 65. The apparatus according to inventive concept 64, wherein the non-flat mirror is concave.

There is additionally provided in accordance with an inventive concept 66, apparatus for detecting a concentration of an analyte in a subject, the apparatus configured to be implanted in a body of the subject and comprising:
an optical waveguide having a first end and a second end;
a sensing unit disposed at the first end of the optical waveguide and configured to detect the analyte, the sensing unit comprising:
   at least an inner axial portion, without cells therein, disposed adjacent to the first end of the optical waveguide; and
   at least one chamber adjacent to the inner axial portion, coaxial with the optical waveguide and the inner axial portion, and comprising live cells that are genetically engineered to produce, in a body of the subject, a sensor protein having a binding site for the analyte, the live cells being configured to secrete the sensor protein.

Inventive concept 67. The apparatus according to inventive concept 66, wherein the analyte is glucose.

Inventive concept 68. The apparatus according to inventive concept 66, wherein the optical waveguide comprises an optical fiber.

Inventive concept 69. The apparatus according to inventive concept 66, wherein the optical waveguide comprises a planar optical waveguide.

There is yet additionally provided in accordance with an inventive concept 70, apparatus for detecting a concentration of an analyte in a subject, the apparatus configured to be implanted in a body of the subject and comprising:
an optical waveguide;
a chamber surrounding a distal portion of the optical waveguide, the distal portion of the optical waveguide extending along at least 75% of a length of the chamber; and
live cells that are genetically engineered to produce, in a body of the subject, a sensor protein having a binding site for the analyte, the live cells being disposed within the chamber.

Inventive concept 71. The apparatus according to inventive concept 70, wherein the analyte is glucose.

Inventive concept 72. The apparatus according to inventive concept 70, wherein the distal portion of the optical waveguide has a distal-portion diameter that is smaller than a proximal-portion diameter of a proximal portion of the optical waveguide.

Inventive concept 73. The apparatus according to inventive concept 72, wherein the proximal portion diameter is equal to a combined diameter of the chamber and the distal portion of the optical waveguide.

Inventive concept 74. The apparatus according to inventive concept 70, wherein the optical waveguide comprises an optical fiber.

Inventive concept 75. The apparatus according to inventive concept 70, wherein the optical waveguide comprises a planar optical waveguide.

There is further provided in accordance with an inventive concept 76, apparatus for detecting a concentration of an analyte in a subject, the apparatus configured to be implanted in a body of the subject and comprising:
an optical waveguide configured to transmit excitation light;
a chamber comprising live cells that are genetically engineered to produce, in a body of the subject, a fluorescent sensor protein having a binding site for the analyte, the fluorescent sensor protein being configured to transmit fluorescent light in response to the excitation light, the chamber being disposed coaxially with respect to the optical waveguide;
a lens disposed between the optical waveguide and the chamber, the lens being configured to focus light from the optical waveguide to the chamber and light from the chamber to the optical waveguide;
a first mirror, optically coupled to the chamber and disposed between a proximal end of the chamber and the lens, the first mirror configured to reflect the excitation light within the chamber and transmit the fluorescent light from within the chamber toward the lens and the optical waveguide, the first mirror being shaped to define a pinhole configured to allow passage of the excitation light from the lens into the chamber; and
a second mirror, optically coupled to the chamber and disposed at a distal end of the chamber.

Inventive concept 77. The apparatus according to inventive concept 76, wherein the analyte is glucose.

Inventive concept 78. The apparatus according to inventive concept 76, wherein the first mirror comprises a dichroic mirror.

Inventive concept 79. The apparatus according to inventive concept 76, wherein the optical waveguide comprises an optical fiber.

Inventive concept 80. The apparatus according to inventive concept 76, wherein the optical waveguide comprises a planar optical waveguide.

There is also provided in accordance with an inventive concept 81, a method, comprising:
facilitating measuring of a concentration of an analyte in a body of a subject, from a subcutaneous location of the subject;
measuring a temperature of the subcutaneous location in conjunction with the facilitating of the measuring of the concentration of the analyte; and
calibrating the measurement of the concentration of the analyte in response to the measured temperature.

Inventive concept 82. The method according to inventive concept 81, wherein facilitating the measuring comprises subcutaneously implanting a device configured to measure the analyte, and wherein the method further comprises calibrating the device prior to the measuring of the concentration of the analyte.

Inventive concept 83. The apparatus according to inventive concept 81, wherein the analyte is glucose.

The present invention will be more fully understood from the following detailed description of some applications thereof, taken together with the drawings, in which:

### BRIEF DESCRIPTION OF THE DRAWINGS

Fig. 1 is a schematic cross-sectional illustration of apparatus for facilitating cell growth, in accordance with some applications of the present invention;
Fig. 2A is a schematic cross-sectional illustration of apparatus for facilitating cell growth comprising a membrane structure, in accordance with some applications of the present invention;
Fig. 2B is a schematic cross-sectional illustration of apparatus for facilitating cell growth comprising a membrane structure, in accordance with some applications of the present invention;
Fig. 3A is a three-dimensional schematic illustration of apparatus for facilitating cell growth, in accordance with some applications of the present invention;
Fig. 3B is a three-dimensional schematic illustration of the apparatus of Figs. 1, 2A, and 2B, in accordance with some applications of the present invention;
Fig. 4 is a schematic cross-sectional illustration of apparatus for facilitating cell growth, in accordance with some applications of the present invention;
Fig. 5 is a schematic block diagram of a two-chamber configuration of apparatus for facilitating cell growth, in accordance with some applications of the present invention;
Fig. 6A is a schematic cross-sectional illustration of a sensing unit comprising an internal protein chamber and an external cell chamber, in accordance with some applications of the present invention;
Fig. 6B is a schematic cross-sectional illustration of a sensing unit comprising an internal cell chamber and an external protein chamber, in accordance with some applications of the present invention;
Fig. 7A is a schematic cross-sectional illustration of a sensing unit comprising a cell chamber and a protein chamber, in accordance with some applications of the present invention;
Fig. 7B is a schematic cross-sectional illustration of a sensing unit comprising an inner axial portion without cells, and comprising an external chamber having cells and protein, in accordance with some applications of the present invention;
Fig. 8 is a schematic cross-sectional illustration of a sensing unit comprising a transparent inner axial portion that is coaxial with an external cell chamber, in accordance with some applications of the present invention;
Fig. 9A is a schematic cross-sectional illustration of a sensing unit comprising an optical waveguide surround by a cell chamber, in accordance with some applications of the present invention;
Fig. 9B is a schematic cross-sectional illustration of a sensing unit comprising an optical waveguide of variable diameter, in accordance with some applications of the present invention;
Fig. 10A is a schematic cross-sectional illustration of a sensing unit, in accordance with some applications of the present invention;
Fig. 10B is a schematic cross-sectional illustration of a sensing unit comprising a non-flat mirror at the distal end of the sensing unit, in accordance with some applications of the present invention;
Fig. 11A is a schematic cross-sectional illustration of a sensing unit having a resonant cavity formed between two mirrors, in accordance with some applications of the present invention;
Fig. 11B is a schematic cross-sectional illustration of a sensing unit surrounded at least in part by a protective layer, in accordance with some applications of the present invention;
Fig. 12 is a schematic cross-sectional illustration of a sensing unit comprising a planar optical waveguide, in accordance with some applications of the present invention;
Figs. 13A-B are schematic cross-sectional illustrations of additional configurations of sensing units, in accordance with respective applications of the present invention;
Figs. 14A-B are graphs showing the measurement of glucose in accordance with an experiment conducted by the inventors;
Fig. 15 is a schematic cross-sectional diagram of a three-layer cell encapsulation structure, in accordance with an application of the present invention;
Figs. 16A-B show dynamics of cell populations in an experiment conducted by the inventors;
Figs. 17A-B shows the results of an intensity analysis performed in the experiment of Figs. 16A-B;
Fig. 18 shows PrestoBlue metabolism tests performed during the experiment of Figs. 16A-B;
Fig. 19 is a schematic cross-sectional illustration of a multi-layer immunoisolation system, in accordance with an application of the present invention;
Fig. 20 is a schematic cross-sectional illustration of a multi-layer immunoisolation system, in accordance with an application of the present invention; and
Fig. 21 is a schematic cross-sectional illustration of another multi-layer immunoisolation system, in accordance with an application of the present invention.

### DETAILED DESCRIPTION OF APPLICATIONS OF THE INVENTION

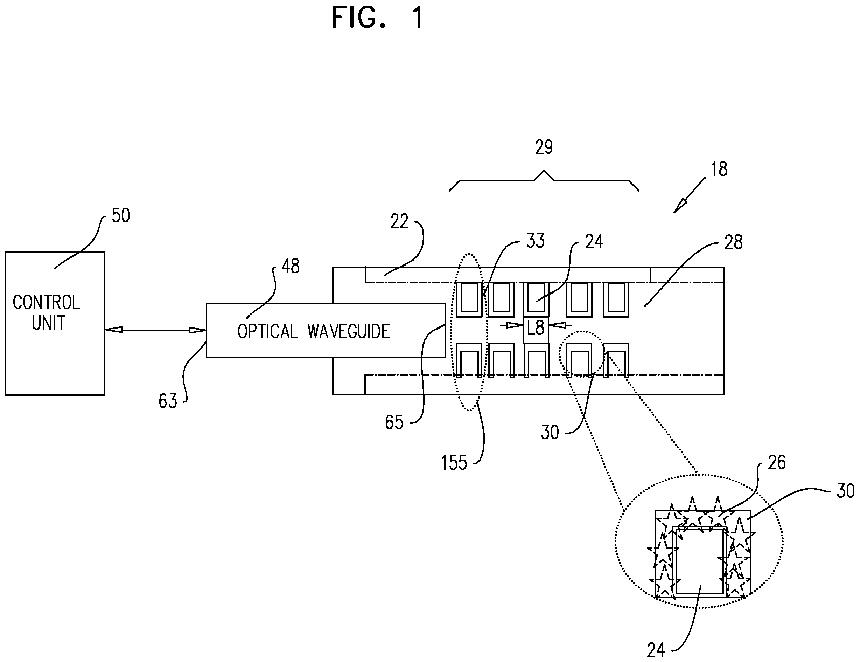

Fig. 1 is a schematic cross-sectional illustration of implantable apparatus 18 for facilitating cell growth, in accordance with some applications of the present invention. A scaffold material 28 is shaped to define one or more chambers 155, e.g., implemented as one or more wells 30. Scaffold material 28 is typically but not necessarily cylindrical, e.g., right-circular-cylindrical. Cells 26, such as one or more monolayers of cells 26, are disposed in wells 30 and/or elsewhere on scaffold material 28. The one or more wells can be a plurality of wells, as shown in Fig. 1, or can be a single well (e.g., shaped to define a helix, like a screw-thread). A total surface area of scaffold material 28 upon which the cells are disposed is typically at least 60% of a total surface area of the scaffold which is illuminated when light passes through the optical waveguide.

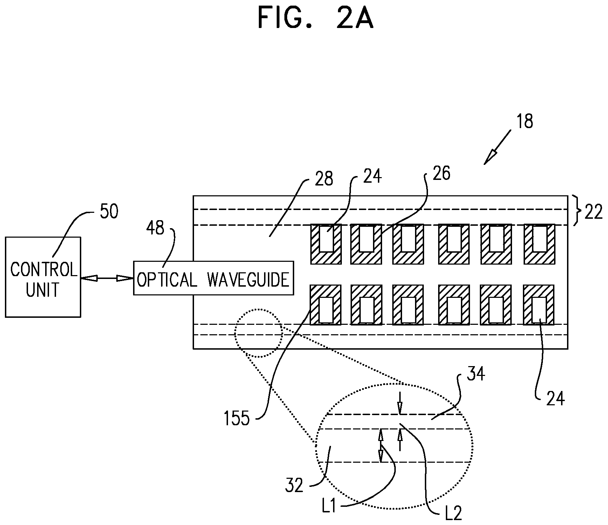

A membrane structure 22 permeable to nutrients surrounds scaffold material 28 at least in part and is mechanically supported by the scaffold material. Membrane structure 22 may be a simple membrane (e.g., a homogeneous membrane), or a membrane having multiple components, such as a spatially non-homogeneous membrane structure (e.g., as described hereinbelow with reference to Figs. 2A-B).

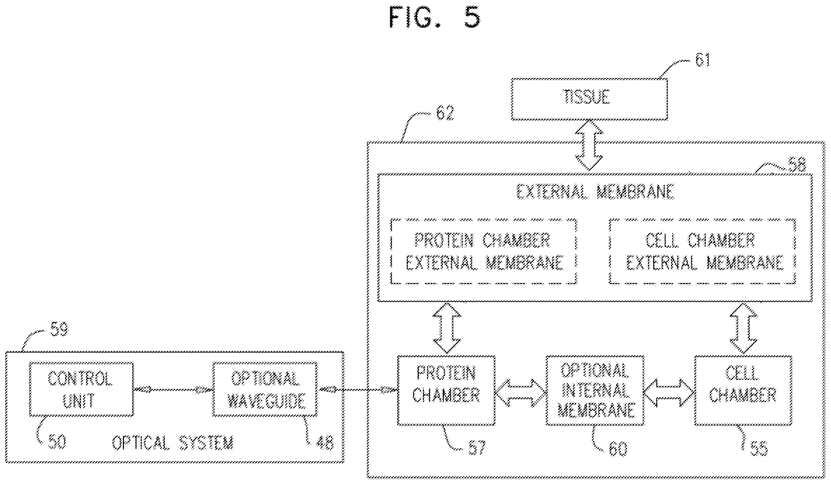

For some applications, an optical system (e.g., optical system 59 as indicated in Fig. 5) comprises an optical waveguide 48, which is optically coupled to scaffold material 28 (e.g., at least partially disposed within the scaffold material), in order to enable transmission of an optical signal to and from a control unit 50 of the optical system. For some applications, optical waveguide 48 comprises an optical fiber.

In general, in applications described herein with reference to all of the figures, the cells secrete a sensor protein. Alternatively, the cells express but do not secrete a sensor protein.

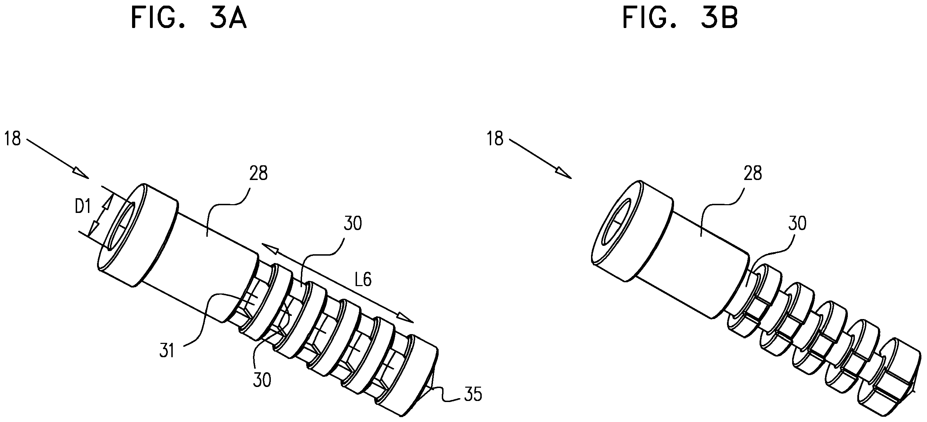

In accordance with some applications of the present invention, the diameter of a part of the scaffold material 28 in which optical waveguide 48 is inserted is about 300-600 microns (e.g., 500 microns), and the waveguide itself typically has a diameter of 300-600 microns (e.g., 500 microns). The cells are typically disposed only on a portion 29 of scaffold material 28 that is shorter than the entire length of the scaffold material. Portion 29 is typically 1-10 mm (e.g., 2-4 mm) in length. Although four rings 33 of scaffold material 28 defining wells 30 are shown in Fig. 1 (as well as in Figs. 3A-B), other applications may include a different number of rings (e.g., five rings, as shown in Figs. 2A-B, or in general 2-10 rings). The gaps between the rings L8 are typically between 0.5 and 1 mm.

In accordance with some applications of the present invention, cells 26 are genetically engineered to produce, *in situ,* sensor protein (not shown) comprising a fluorescent protein donor (e.g., cyan fluorescent protein (CFP)), a fluorescent protein acceptor (e.g., yellow fluorescent protein (YFP)), and a binding site (e.g., glucose-galactose binding site) for an analyte. When the protein binds an analyte such as glucose, binding of the glucose causes a conformational change in the sensor protein and a corresponding changing in the distance between respective donors and acceptors. Fluorescence resonance energy transfer (FRET) involves the transfer of energy from an excited fluorophore (the donor) to another fluorophore (the acceptor) when the donor and acceptor molecules are in close proximity to each other. FRET enables the determination of the relative proximity of the molecules for investigating, for example, the binding of analyte, and thus the concentration of the analyte. All of the apparatus and methods described herein, with reference to each of the figures, may be combined with techniques described in the above-referenced PCT Patent Application Publication WO 2006/006166 to Gross et al. and US Patent 7,951,357 filed in the national stage thereof, and in US 2010/0202966 to Gross et al.

Typically, scaffold material 28 is optically transparent. Scaffold material 28 may comprise, for example, molded plastic or polystyrene. Excitation light generated by control unit 50 passes through optical waveguide 48, and enters each well 30 via transparent scaffold material 28. A signal of light of different wavelengths emitted by the sensor proteins is passed by the optical waveguide 48 to control unit 50. Control unit 50 interprets the different wavelengths in the received light signal as indicative of which portion of the sensor proteins have undergone the conformational change, and, therefore, of the concentration of the analyte (e.g., glucose). Typically, scaffold material 28 is rigid.

For some applications, the scaffold is fabricated using 3D printing, and may comprise a biocompatible material, such as MED610.

Fig. 2A is a schematic cross-sectional illustration of apparatus 18, in accordance with some applications of the present invention. Apparatus 18 as shown in Fig. 2A is the same as apparatus 18 as shown in Fig. 1 and described with reference thereto, except for particular details of membrane structure 22. Membrane structure 22 in the implementation shown in Fig. 2A comprises (a) a first material 32 of the membrane comprising a biodegradable material (such as a hydrogel), and (b) a second material 34 of the membrane comprising a non-biodegradable material. Materials 32 and 34 may be in any suitable geometrical configuration with respect to each other that provides fluid communication between body fluid of the subject and materials 32 and 34. For example, as shown in Fig. 2A, first material 32 is disposed in a first layer that starts out at a thickness L1 of 50-500 um. The molecular weight cutoff (MWCO) of first material 32 is typically less than 100 kilodaltons, or less than 50 kilodaltons. First material 32 is degraded in the body over a period of time (e.g., within a period of two weeks to six months, in the presence of body fluids), such that the MWCO of membrane structure 22 increases over time. The thickness L2 of a second layer, comprising second material 34, may be greater than, less than, or the same as thickness L1 of first material 32. For example, L2 may be at least 50 um and/or less than 250 um. Second material 34 of the membrane structure typically comprises a material such as Polysulfone (PSU), Teflon (pTFE), or polyethersulfone (PES). The second layer is typically but not necessarily disposed between the cells and the first layer.

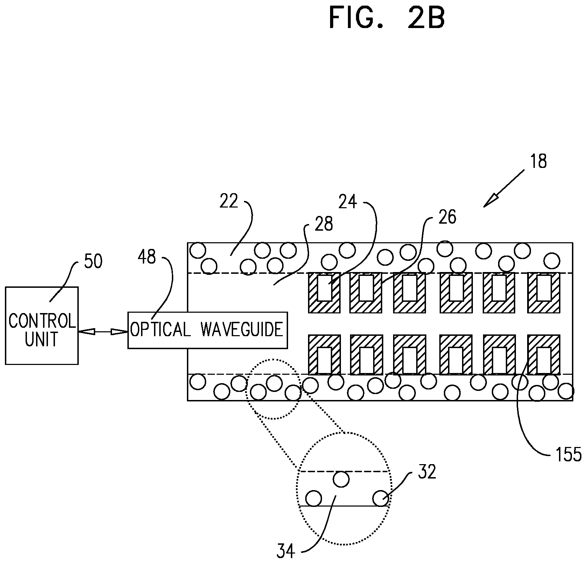

Fig. 2B is a schematic cross-sectional illustration of apparatus 18 for facilitating cell growth, in accordance with some applications of the present invention. Apparatus 18 as shown in Fig. 2B is the same as apparatus 18 as shown in Figs. 1 and 2A and described with reference thereto, except for particular details of membrane structure 22 described hereinbelow. Membrane structure 22 surrounds scaffold material 28 at least in part and is permeable to nutrients. Membrane structure 22 in the implementation shown in Fig. 2B comprises a first material 32 comprising a biodegradable material, and a second material 34 comprising a non-biodegradable material. Second material 34 is impregnated with first material 32. In some applications, membrane structure 22 comprises a non-biodegradable material shaped to define a plurality of holes. The non-biodegradable material is impregnated with a biodegradable solution. In applications in which the biodegradable solution comprises alginate, the solution may be fixed for example by exposing the alginate to ions (e.g., calcium, strontium, or barium ions). In applications in which the biodegradable solution comprises Poly(ethylene glycol) (PEG), ultraviolet light may be used to fix the solution. In some applications, the holes in the non-biodegradable membrane are permeable to molecules less than 600 kilodaltons and/or impermeable to molecules greater than 600 kilodaltons. For example, the non-biodegradable membrane may have a molecular weight cutoff (MWCO) which is under 100 kilodaltons. In some applications, the holes in the non-biodegradable membrane are permeable to molecules less than 300 kilodaltons and/or impermeable to molecules greater than 300 kilodaltons. In some applications, the holes in the non-biodegradable membrane are permeable to molecules that are 80-300 kilodaltons.

Reference is now made to Figs. 2A and 2B. In summary, in accordance with the description of these figures, one or more chambers 155 having isolated cells disposed therein are surrounded at least in part by membrane structure 22. Membrane structure 22 in a first state thereof has a first molecular weight cut off (MWCO), which is configured to transition to a second state thereof, in which the membrane structure has a second MWCO, the second MWCO being higher than the first MWCO (e.g., at least three times higher than the first MWCO). It is noted that such a membrane structure is useful both in the context of apparatus for analyte sensing, as generally described herein, as well as in general, in the context of implantable apparatus for maintaining transplanted cells (for example, without a light source and/or without a control unit).

For example, the first MWCO may be less than 150 kilodaltons (e.g., less than 100 kilodaltons, e.g., less than 50 kilodaltons), while the second MWCO is typically greater than 150 kilodaltons. In a particular example, the first MWCO is less than 100 kilodaltons and the second MWCO is greater than two times the first MWCO.

Typically, membrane structure 22 in the first state is not permeable to IgG, while in the second state structure 22 is permeable to IgG. Alternatively or additionally, membrane structure 22 in the first state is not permeable to transferrin, while in the second state structure 22 is permeable to transferrin. In both states, membrane structure 22 is permeable to glucose, and not permeable to white blood cells.

The transition from the first state to the second state may be achieved (as described hereinabove with reference to Figs. 2A and 2B) by membrane structure 22 comprising (a) first material 32 that is biodegradable and has the first MWCO, and (b) second material 34, that is non-biodegradable and has the second MWCO. The second material is typically permeable to molecules that are at least 80 kilodaltons, e.g., molecules that are at least 300 kilodaltons.

Fig. 3A is a schematic illustration of apparatus 18 for facilitating cell growth, in accordance with some applications of the present invention. In this three-dimensional representation of apparatus 18 from Figs. 1, 2A, and 2B, optically transparent scaffold 42 supports wells 30 configured for facilitating cell growth. Unlike the apparatus shown in Figs. 1 and 2A-B, apparatus 18 shown in Fig. 3A provides a plurality of surfaces 31 perpendicular to and facing optical waveguide 48. Cells 26, such as a monolayer of cells 26, are disposed on surfaces 31, typically in addition to cells 26 disposed in wells 30 as described hereinabove with reference to Figs. 1 and 2A-B. Aside from this difference, apparatus and techniques described hereinabove with reference to Figs. 1 and 2A-B apply to Fig. 3A as well.

Reference is now made to Figs. 1, 2A, 2B, 3A, and 3B. Typically, a substantial amount of surface area is provided for cell growth in wells 30 and/or on surfaces 31, this surface area being located close to a distal tip of optical waveguide 48, and within a fairly narrow exit cone of light leaving (and entering) the waveguide. For example, as shown in Fig. 3A, when optical waveguide 48 comprises an optical fiber having a diameter D1, scaffold 42 provides within a 22 degree exit cone from the distal tip of the optical waveguide, within a distance that is four times D1 (marked L6) from the distal tip of waveguide 48, a surface area of the scaffold of at least 4 * pi * (D1 / 2) ^ 2 upon which the cells are disposed. In some applications, the scaffold is 2-4 mm in length, and/or the scaffold volume is 0.5-2 microliter (ul). The surface area of the portion of the scaffold upon which cells are disposed is typically 2-4 mm^2 (e.g., 3 mm^2).

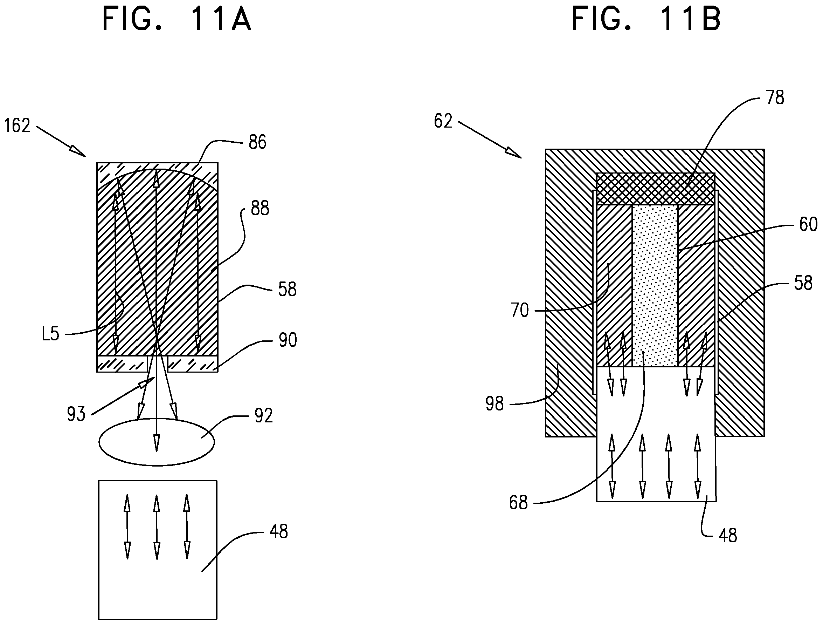

For some applications, a mirror 35 (e.g., a non-flat mirror, such as a concave mirror) is disposed at the distal end of apparatus 18, in order to reflect light back toward the sensor protein and toward optical waveguide 48. Use of such a mirror is shown in Figs. 3A, 3B, and 11A. (Alternatively, e.g., in Fig. 3A, the mirror is flat.) For some applications, a corresponding non-flat or flat mirror 35 is used, mutatis mutandis, with the apparatus shown in Figs. 1, 2A, or 2B.

Fig. 3B is a schematic illustration of apparatus 18 for facilitating cell growth, in accordance with some applications of the present invention. In this three-dimensional representation of apparatus 18 from Figs. 1, 2A, and 2B, optically transparent scaffold 42 supports wells 30 configured for cell growth. The apparatus of Fig. 3B differs from that of Fig. 3A in that it does not provide the plurality of surfaces 31.

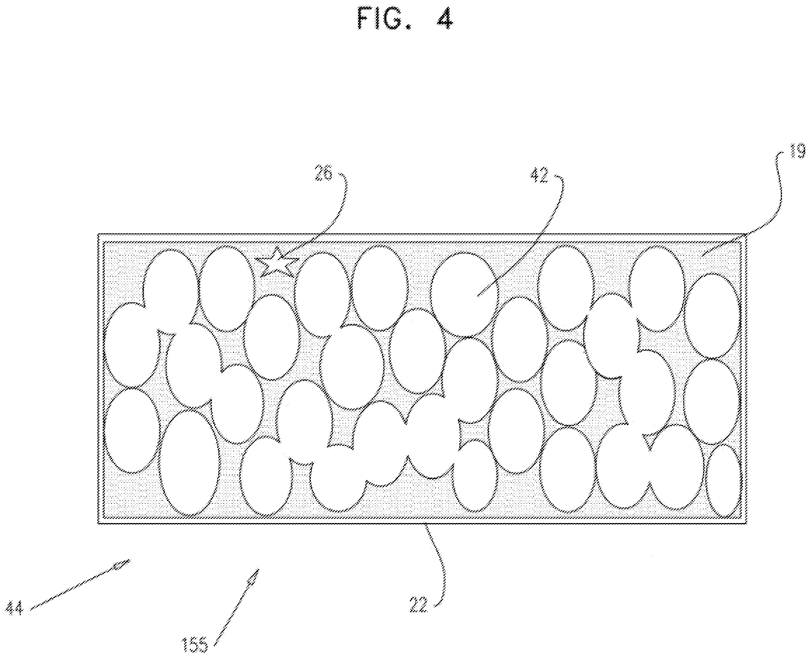

Fig. 4 is a schematic cross-sectional illustration of apparatus 44 for facilitating cell growth, in accordance with some applications of the present invention. Apparatus 44 comprises a chamber 155 for containing the cells, and is typically used in combination with an optical waveguide 48, a control unit 50, and a membrane structure 22, as described with reference to the other figures. For some applications, (a) an optical waveguide is not utilized, or (b) an optical waveguide and a control unit are not utilized. In the depicted application, a scaffold 19 conducive to cell growth (e.g., comprising a hydrogel) typically has at least 1,000 cells 26 (e.g., at least 2,000 cells 26 or at least 5,000 cells 26) and/or less than 30,000 cells 26 (e.g., less than 20,000 cells or less than 10,000 cells 26) disposed therein. Scaffold 19 is typically but not necessarily optically transparent. Typically, the density of the cells is at least 10 million cells/mL and/or less than 30 million cells/mL. A typical volume in which the cells are contained is at least 0.2 microliters and/or less than 2 microliters, e.g., at least 0.5 microliters and/or less than 1 microliter.

At least one nutrient supply compartment comprising a nutrient permeable medium 42 that is arranged to not be conducive to cell growth therein is interspersed with scaffold 19, such that at least 80% of the cells within scaffold 19 are disposed within 100 um (e.g., within 50 um) of nutrient permeable medium 42. The nutrient permeable medium is positioned such that an easy diffusion path for nutrients is thus provided, by the nutrient permeable medium, between the subject's body and the at least 80% of the cells.

A volume of the nutrient supply compartment comprising nutrient permeable medium 42 is typically 25%-75% of a volume of chamber 155. Typically, nutrient permeable medium 42 comprises a hydrogel, but in general may comprise any material which suitably diffuses nutrients. The nutrient permeable medium may alternatively or additionally comprises one or more materials such as silicone rubber, fused glass powder, sintered glass powder, a hydrogel, and/or an alginate. This material may be shaped to define one or more spheres, e.g., at least 100 and/or less than 1000 spheres. The volume of chamber 155 is typically at least 20 times (e.g., at least 100 times, e.g., 200-1000 times) a volume of at least one of the spheres. For some applications, the spheres are disposed in the chamber in an efficient packing configuration.

Fig. 5 is a schematic block diagram of a two-chamber sensing unit 62 in optical communication with an optical system 59, in accordance with some applications of the present invention. A first cell chamber 55 contains cells and a second sensor protein chamber 57, which is transparent, contains a sensor protein secreted by the cells. Sensing unit 62 is configured to allow the sensor protein to diffuse from cell chamber 55 to sensor protein chamber 57, e.g., through an optional internal membrane 60. As a result, cell chamber 55 contains the cells, cytosolic sensor protein, and sensor protein secreted from the cells, while sensor protein chamber 57 contains secreted sensor protein but not cells. The transparency of the sensor protein chamber enables the optical measurement of the fluorescence spectrum, via an optional optical waveguide 48 of optical system 59, and thus facilitates a measure of a level of an analyte in the subject's body. For some applications, cell chamber 55 is not optically transparent. Alternatively or additionally, for some applications, optional internal membrane 60 is not optically transparent. The non-transparency of cell chamber 55 and/or internal membrane 60 helps optically insulate the sensor protein in cell chamber 55 from optical waveguide 48, which may increase the quality of the optical signal, as described Optical waveguide 48 is optically coupled to sensor protein chamber 57, and carries light between protein chamber 57 and control unit 50, as described hereinabove.

It is noted that (as shown in Fig. 5) an optical waveguide is optional, in the context of the present application (including all listed embodiments, inventive concepts, and figures) and in the techniques recited in the claims. Thus, for example, for each independent claim and inventive concept of the present application which recites an optical waveguide, the scope of the present invention includes a corresponding implementation in which an optical waveguide is not utilized. In such implementations, light is conveyed between the optical system (e.g., optical system 59) and the protein via other means. For example, the optical system may be placed outside of the subject's body, and light is conveyed between the optical system and the protein transcutaneously.

The glucose level and the time response to glucose changes that the sensor protein experiences while still inside the cells depends on the uptake dynamics of glucose into the cells. This dynamic adds complexity to the sensing mechanism. This complexity does not exist when the sensor protein is secreted from the cells and can react with the glucose as soon as the glucose enters the device. Accordingly, the two optical signals obtained by reading the fluorescence from cytosolic sensor protein and from free secreted sensor protein have different time responses and different calibration factors. For improved accuracy, some applications of the present invention reduce mixing of these two optical signals. Providing transparent sensor chamber 57 and non-optically-transparent cell chamber 55 enables the optical signal to be obtained primarily or exclusively from the free secreted sensor protein, rather than the cytosolic sensor protein. Alternatively or additionally, optical waveguide 48 is positioned so as to reduce the amount of light passing therethrough that includes fluorescence generated within cell chamber 55.

For some applications, cell chamber 55 and sensor protein chamber 57 are separated by internal membrane 60, which has a molecular weight cutoff sufficiently large to allow the sensor protein to freely diffuse between the two chambers while preventing cells from crossing between the chambers. Therefore, for typical applications in which the sensor protein has a size of 90 KDa and cells cannot pass through a membrane with pore size of 1 um or less, the inner membrane typically has a MWCO which is larger than 50 kDa (e.g., larger than 90 KDa) and a pore size smaller than about 1 um.

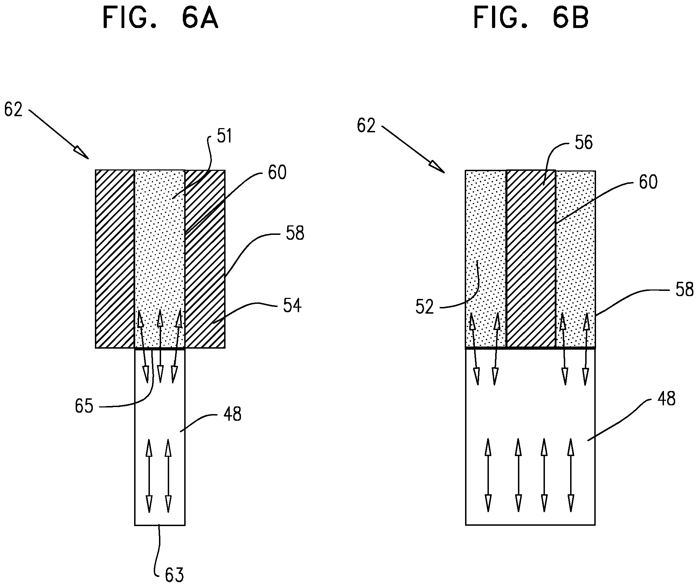

Alternatively, for other applications, internal membrane 60 is not provided, and separation between the cells of cell chamber 55 and the free sensor protein of sensor protein chamber 57 is provided by the two chambers comprising different materials. The cell chamber typically comprises a material that supports cell growth, and optionally also allows cell attachment. For example, the materials of the cell chamber may include sponge-like structures formed by dehydrated alginate; randomly-scattered fibers, e.g., created by electro-spinning, the fibers typically comprising plastic types which enable cell attachment and are optionally plasma-treated for enhanced surface charge; and/or solid structures comprising, for example, collagen, fibrinogen, or other proteins present in the external cellular matrix (ECM) of cells. The sensor protein chamber is filled with an optically-transparent material that does not allow cell proliferation but does allow free diffusion of the fluorescent biosensor, typically a hydrogel, e.g., alginate or Poly(ethylene glycol) (PEG). These separation techniques may be used instead of or in addition to internal membrane 60 in any of the configurations described hereinbelow with reference to Figs. 6A-B, 7A-B, 8, 9A-B, 10A-B, 11A-B, 12, and 13A-B.

Typically, but not necessarily, sensing unit 62 further comprises an external membrane 58, which surrounds all or a part of the sensing unit, and thus provides an interface between the sensing unit and tissue 61 of the subject. External membrane 58 is configured to prevent the sensor protein from escaping the sensing unit (both from cell chamber 55 and sensor protein chamber 57), while maintaining ample diffusion of nutrients to the chambers. Typically, membranes effectively block the escape of molecules which are at least three times the rated MWCO. Thus for a sensor protein of a typical size of 90 KDa to be maintained long-term in the sensing unit, the MWCO of external membrane 58 should be no greater than 30 KDa. On the other hand, in order to maintain diffusion of nutrients into the sensing unit, the MWCO of the external membrane should be no less than a few KDa. Therefore, the typical MWCO of the outer membrane is in between 3 KDa and 30 KDa.

As appropriate for various applications of the present invention, membrane 58 may be a single membrane, surrounding both cell chamber 55 and sensor protein chamber 57. Alternatively, a membrane may surround cell chamber 55 while another membrane surrounds sensor protein chamber 57, each of these membranes separating the respective chambers 55 and 57 from tissue 61.

Fig. 6A is a schematic cross-sectional illustration of a sensing unit 62 for detecting a concentration of an analyte and configured to be implanted in a body of a subject, in accordance with some applications of the present invention. In this configuration, sensing unit 62 comprises an internal sensor protein chamber 51 and an external cell chamber 54. Internal sensor protein chamber 51 is one implementation of sensor protein chamber 57, and external cell chamber 54 is one implementation of cell chamber 55, both described hereinabove with reference to Fig. 5. Optionally, internal membrane 60 separates internal sensor protein chamber 51 from external cell chamber 54. External membrane 58 surrounds external cell chamber 54, at least in part. Optical waveguide 48, having a proximal end 63 and a distal end 65, is typically disposed parallel to a longitudinal axis of internal sensor protein chamber 51, in order to enable transmission of light from optical waveguide 48 to the sensor protein in internal sensor protein chamber 51 that was produced by cells in external cell chamber 54. In addition, optical waveguide 48 carries light from internal sensor protein chamber 51 to control unit 50 (e.g., control unit 50 as shown in Figs. 1-2B), to allow control unit 50 to analyze the wavelengths in the received light and identify an indication of the level of the analyte (e.g., blood glucose level). (The arrows in Fig. 6A schematically represent light. Unless otherwise mentioned, light rays represent both excitation light and collected fluorescence.)

Fig. 6B is a schematic cross-sectional illustration of another configuration of sensing unit 62, in accordance with some applications of the present invention. (Except for differences as described hereinbelow, the configuration shown in Fig. 6B is generally similar to that described hereinabove with reference to Fig. 6A.) In this configuration, sensing unit 62 comprises internal cell chamber 56 and external sensor protein chamber 52. External sensor protein chamber 52 is one implementation of sensor protein chamber 57, and internal cell chamber 56 is one implementation of cell chamber 55, both described hereinabove with reference to Fig. 5. Optionally, internal membrane 60 separates internal cell chamber 56 from external sensor protein chamber 52. External membrane 58 surrounds external sensor protein chamber 52. Optical waveguide 48 is typically disposed parallel to a longitudinal axis of external sensor protein chamber 52.

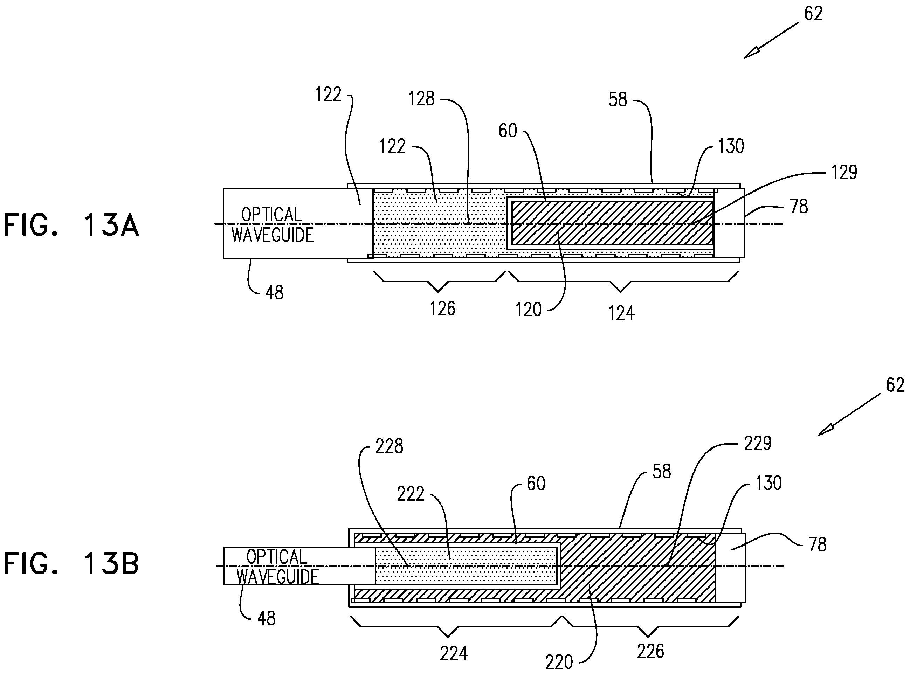

In the configurations described with reference to Figs. 6A-B, as well as with reference to Fig. 13, sensing unit 62 is typically cylindrical, e.g., right-circular-cylindrical.

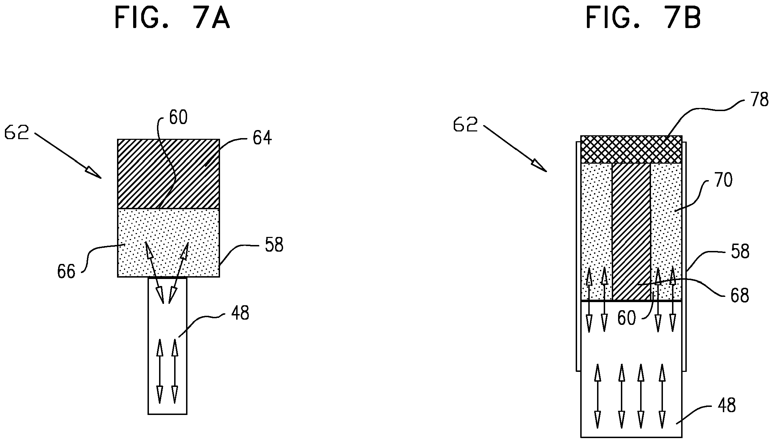

Fig. 7A is a schematic cross-sectional illustration of yet another configuration of sensing unit 62, in accordance with some applications of the present invention. (Except for differences as described hereinbelow, the configuration shown in Fig. 7A is generally similar to that described hereinabove with reference to Fig. 6A.) In this configuration, sensing unit 62 comprises a cell chamber 64 separated by optional internal membrane 60 from a sensor protein chamber 66. Sensor protein chamber 66 is one implementation of sensor protein chamber 57, and cell chamber 64 is one implementation of cell chamber 55, both described hereinabove with reference to Fig. 5. An external membrane 58 surrounds both the cell chamber 64 and the sensor protein chamber 66, at least in part. Optical waveguide 48 is typically disposed parallel to a longitudinal axis of sensor protein chamber 66, and such that sensor protein chamber 66 is between cell chamber 64 and optical waveguide 48.

Fig. 7B is a schematic cross-sectional illustration of still another configuration of sensing unit 62, in accordance with some applications of the present invention. (Except for differences as described hereinbelow, the configuration shown in Fig. 7B is generally similar to that described hereinabove with reference to Fig. 6A.) In this configuration, sensing unit 62 comprises an inner axial sensor protein chamber 68. Inner axial sensor protein chamber 68 is surrounded by outer cell chamber 70. Inner axial sensor protein chamber 68 is one implementation of sensor protein chamber 57, and outer cell chamber 70 is one implementation of cell chamber 55, both described hereinabove with reference to Fig. 5. Optionally, internal membrane 60 separates the inner axial sensor protein chamber 68 from outer cell chamber 70. External membrane 58 surrounds outer cell chamber 70, at least in part. Optical waveguide 48 is typically disposed parallel to a longitudinal axis of inner axial sensor protein chamber 68 and outer cell chamber 70.

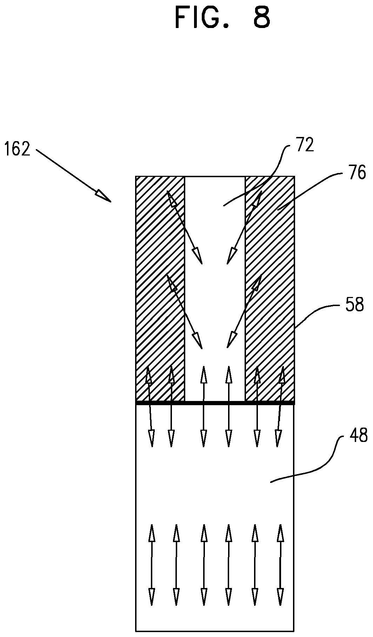

Fig. 8 is a schematic cross-sectional illustration of a sensing unit 162, in accordance with some applications of the present invention. (Except for differences as described hereinbelow, the configuration shown in Fig. 8 is generally similar to that described hereinabove with reference to Fig. 6A.) In this configuration, sensing unit 162 comprises a transparent inner axial portion 72, which is typically solid (e.g., a continuation of optical waveguide 48) and does not permit fluid flow therethrough. Alternatively, portion 72 is for example a hydrogel, and permits fluid flow therethrough. Transparent inner axial portion 72 is surrounded by an outer cell chamber 76, which contains cells (in a like manner to cell chamber 70 described hereinabove), as well as protein produced by the cells (for example, secreted by the cells). External membrane 58 surrounds the outer cell chamber 76, at least in part.

Optical waveguide 48 is typically disposed parallel to a longitudinal axis of transparent inner axial portion 72 and outer cell chamber 76. Optical waveguide 48 for this application typically comprises an optical fiber. Inner axial portion 72 may also effectively be an optical fiber, however unlike many optical fibers, inner axial portion 72 for this application typically does not have a clad around a portion of the lateral surface thereof (as shown) from which it is desired that light escapes (and enters). Therefore, inner axial portion 72 releases light and receives light through its lateral surface, both to and from outer cell chamber 76. Alternatively or additionally, the lateral surface of inner axial portion 72 is roughened (or otherwise treated) in order to enhance the passage of light between inner axial portion 72 and outer cell chamber 76. Further alternatively or additionally, the refractive index of inner axial portion 72 is matched to that of chamber 76, in order to minimize reflections. For some applications (configuration not shown), inner axial portion 72 has a profile different than a simple cylinder, e.g. a cone, thereby increasing the angle between light rays and the normal to the surface.

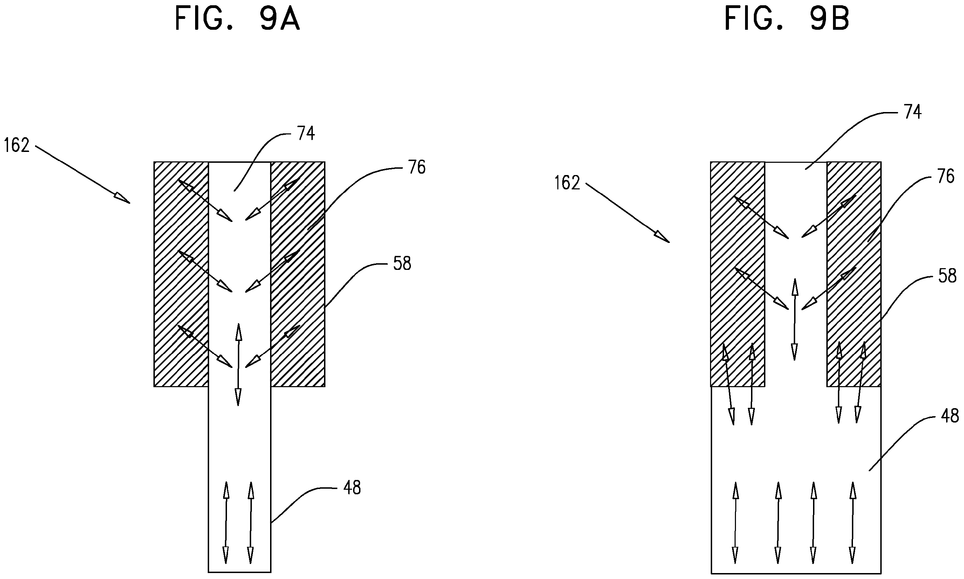

Fig. 9A is a schematic cross-sectional illustration of another configuration of sensing unit 162, in accordance with some applications of the present invention. (Except for differences as described hereinbelow, the application shown in Fig. 9A is generally similar to that described hereinabove with reference to Fig. 8.) In this configuration, optical waveguide 48 of sensing unit 162 typically (but not necessarily) has a constant diameter. Optical waveguide 48 is disposed coaxially and partially within outer cell chamber 76. External membrane 58 at least partially surrounds outer cell chamber 76. Light transmitted from optical waveguide 48 passes through sensor protein in outer cell chamber 76. The light is then transmitted back to control unit 50 (e.g., control unit 50 as shown in Figs. 1-2B).

Fig. 9B is a schematic cross-sectional illustration of still another configuration of sensing unit 162, in accordance with some applications of the present invention. (Except for differences as described hereinbelow, the application shown in Fig. 9B is generally similar to that described hereinabove with reference to Fig. 9A.) In this configuration, optical waveguide 48 of sensing unit 162 has a variable diameter. A distal portion of optical waveguide 48 having a first diameter defines an inner core 74 of outer cell chamber 76, and is contiguous with a proximal portion of optical waveguide 48 having a second diameter greater than the first diameter. Inner core 74 is coaxial with outer cell chamber 76. External membrane 58 at least partially surrounds outer cell chamber 76. Light enters and leaves optical waveguide 48 both via the outer surface of inner core 74, and via the distal-most surface of the proximal portion of optical waveguide 48.

Reference is made to Figs. 9A and 9B. Optical waveguide 48 for these applications typically comprises an optical fiber. However, unlike many optical fibers, the optical fiber for this application typically does not have a clad around a portion of the lateral surface of the fiber (as shown) from which it is desired that light escapes (and enters). Therefore, optical waveguide 48 releases light and receives light through its lateral surface, both to and from outer cell chamber 76. Alternatively or additionally, the lateral surface of the portion of optical waveguide 48 surrounded by chamber 76 is roughened (or otherwise treated) in order to enhance the passage of light between optical waveguide 48 and outer cell chamber 76. Further alternatively or additionally, the refractive index of the portion of optical waveguide 48 surrounded by chamber 76 is matched to that of chamber 76, in order to minimize reflections. For some applications (configuration not shown), the portion of optical waveguide 48 surrounded by chamber 76 has a profile different from a simple cylinder, e.g. a cone, thereby increasing the angle between light rays and the normal to the surface.

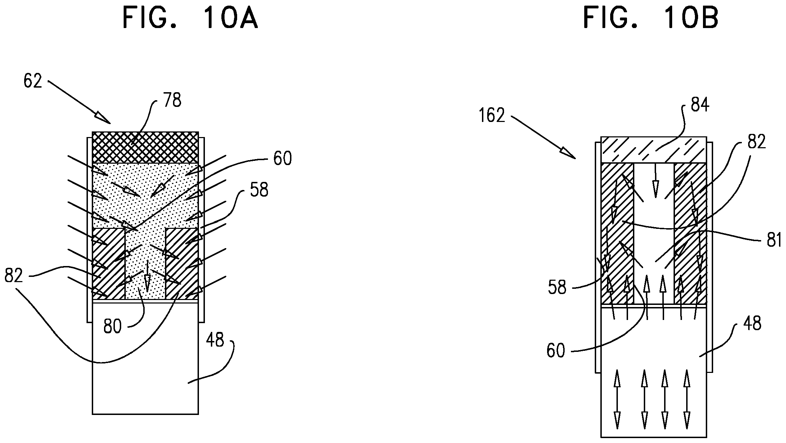

Fig. 10A is a schematic cross-sectional illustration of another configuration of sensing unit 62, in accordance with some applications of the present invention. (Except for differences as described hereinbelow, the configuration shown in Fig. 10A is generally similar to that described hereinabove with reference to Fig. 6A.) In this implementation, diffusion of nutrients is provided via a cell-free zone of sensing unit 62. A distinct protein chamber is not necessarily provided in this implementation.

As shown in Fig. 10A, sensing unit 62 comprises a partially-internal chamber 80 surrounded only in part by an outer cell chamber 82 (which functions like outer cell chamber 76 described hereinabove). A distinct protein chamber is not necessarily provided in this implementation. That is, for some applications the cells do not secrete the protein, and chamber 80 is not a protein chamber. Optionally, internal membrane 60 separates outer cell chamber 82 from partially-internal chamber 80. Outer cell chamber 82 is one implementation of cell chamber 55, described hereinabove with reference to Fig. 5. External membrane 58 surrounds outer cell chamber 82 at least in part and typically has direct contact with a portion of partially-internal chamber 80. Optical waveguide 48 is typically contiguous with partially-internal chamber 80 and/or outer cell chamber 82. A plug 78 closes the distal end of external membrane 58. Arrows schematically show the direction of diffusion of oxygen and other nutrients. It is noted that the application shown in Fig. 10A provides diffusion of nutrients into outer cell chamber 82 through (a) the outer wall of outer cell chamber 82, as well as (b) an inner wall of outer cell chamber 82 and/or a distal wall of outer cell chamber 82.

Fig. 10B is a schematic cross-sectional illustration of yet another configuration of sensing unit 62, in accordance with some applications of the present invention. (Except for differences as described hereinbelow, the application shown in Fig. 10B is generally similar to that described hereinabove with reference to Fig. 8.) A mirror 84 is disposed at the distal end of the sensing unit. Arrows represent excitation light remaining in part within a transparent inner axial portion 81 of the sensing unit, thus increasing optical excitation (pumping), without reducing the collection efficiency of the fluorescent light. Transparent inner axial portion 81 is typically solid, and does not permit fluid flow therethrough. (For some applications, portion 81 is simply the distal end portion of waveguide 48.) It is noted that the mirror described with reference to a sensing unit as shown in Fig. 10B may, alternatively or additionally, be used in combination with the apparatus shown in and described with reference to Figs. 1-3B, 5-12, and 13A-B.

Reference is made to Figs. 8, 9A, 9B, and 10B. As described, these figures show apparatus for detecting a concentration of an analyte in a subject, the apparatus being configured to be implanted in a body of a subject. As shown in these figures, outer cell chambers 76 and 82 surround a distal portion of optical waveguide 48, the distal portion of the optical waveguide extending along at least 75% of a length of chamber 76 and chamber 82. For some applications (e.g., as shown in Figs. 9B and 10B), the distal portion of the optical waveguide has a distal-portion diameter that is smaller than a proximal-portion diameter of a proximal portion of the optical waveguide.

Reference is still made to Figs. 8, 9A, 9B, and 10B. For some applications, in order to enhance the efficiency of light transfer from the inner core of sensing unit 162 to the outer part and back (e.g., in the configurations described with reference to Figs. 8 and 9A-B), one or both of the following design features is utilized:
- the inner core of sensing unit 162 has a profile different from that of a simple right-circular cylinder. For example, the inner core may be shaped as a cone, thereby increasing the angle between light rays and the normal to the surface; and/or
- roughness may be added to the surface of the inner core of sensing unit 162, e.g., by way of a repetitive relief pattern, in order to promote scattering of light traveling along the core into the outer chamber.

Fig. 11A is a schematic cross-sectional illustration of still another configuration of sensing unit 162, in accordance with some applications of the present invention. In this configuration, sensing unit 162 comprises a cell chamber 88 which, like chamber 76 described with reference to Fig. 8, contains both cells and protein. Cell chamber 88 is optically coupled to a first mirror 90. First mirror 90 is disposed at a proximal end of cell chamber 88. A lens 92 is disposed between first mirror 90 and optical waveguide 48. First mirror 90 is configured to reflect excitation light L5 within cell chamber 88 and allow transmission of fluorescent light from within cell chamber 88 toward lens 92 and optical waveguide 48. First mirror 90 is shaped to define a pinhole 93 which allows passage of the excitation light from lens 92 into cell chamber 88. A second mirror 86 is optically coupled to cell chamber 88 and disposed at a distal end of cell chamber 88. External membrane 58 surrounds sensing unit 162. Arrows in the figure represent the excitation light traversing sensing unit 162 in both directions, thus increasing optical excitation (pumping), without reducing the collection efficiency of the fluorescent light.

Fig. 11B is a schematic cross-sectional illustration of another configuration of sensing unit 62, in accordance with some applications of the present invention. (Except for differences as described hereinbelow, the application shown in Fig. 11B is generally similar to that described hereinabove with reference to Fig. 7B.) In this configuration, sensing unit 62 comprises a protective layer 98 that surrounds sensing unit 62 at least in part. Protective layer 98 may be used with the apparatus shown in any of the figures of the present patent application, in accordance with some applications of this invention.

For some applications, protective layer 98 serves one or more of the following functions:
(a) to filter the molecules exchanged between the sensing unit and the tissue, allowing the passage of small molecules, e.g., glucose, but preventing larger molecules, e.g., molecules of the immune system (e.g. IgG); and/or
(b) to minimize the encapsulation of the device by the body. It is known that a fibrination layer typically encapsulates external solid bodies entering a patient's body within a few weeks. For an implanted cell-based glucose sensor, this would possibly reduce the diffusion of nutrients and glucose into the device. One of the purposes of protective layer 98 is to minimize this effect, e.g., by presenting to the body a soft, biodegradable layer.

In order to achieve one or both of the above purposes, protective layer 98 may comprise, for example, a hydrogel (e.g., the hydrogel comprising either synthetic polymers (e.g. Poly(ethylene glycol) (PEG), Poly(ethylene oxide) (PEO), Poly(propylene oxide) (PPO), poly(hydroxyethyl methacrylate) (pHEMA)) or polymers based on proteins (e.g. fibrinogen, collagen), or a combination of both synthetic and protein-based polymers.

For some applications, protective layer 98 experiences minimal fouling while in the body, i.e., pores of layer 98 remain open and are not clogged with various molecules (e.g. proteins).

Reference is made to Figs. 6A, 6B, 7B, 10A, and 11B. It is noted that sensing unit 62 includes at least a first chamber, and at least a second chamber disposed around the first chamber, as shown and described hereinabove. The live cells that are genetically engineered to produce the sensor protein are disposed within the first chamber or within the second chamber.

For some applications, the second chamber is disposed around only a proximal end portion of the first chamber (as shown in Fig. 10A). For applications in which the first chamber contains the live cells and the second chamber surrounds only the proximal portion of the first chamber, the second chamber may facilitate passage of nutrients to the cells in the first chamber from fluid of the subject, by allowing passage of the nutrients through the second chamber to the first chamber, and/or by not impeding passage to the distal portion of the first chamber (which is not surrounded by the second chamber).

As noted, the second chamber may surround substantially the whole length of the first chamber (as shown in Figs. 6A, 6B, 7B, and 11B), e.g., by completely surrounding the first chamber. For applications in which the first chamber contains the live cells and the second chamber surrounds substantially the whole length of the first chamber, the second chamber may facilitate passage of nutrients to the cells in the first chamber from fluid of the subject, by allowing passage of the nutrients through the second chamber to the first chamber.

For some applications, optical waveguide 48 has a diameter that is equal to a diameter of the first chamber (e.g., as shown in Fig. 6A). Alternatively, the optical waveguide has a diameter that is equal to an outer diameter of the second chamber (e.g., as shown in Figs. 6B, 7B, 10A, and 11B).

Optional internal membrane 60 of sensing unit 62 is typically semi-permeable, configured to facilitate passage of the sensor protein from the chamber containing the cells (i.e., the first or the second chamber) to the other chamber (i.e., the second or the first chamber, respectively), while restricting passage of cells through membrane 60.

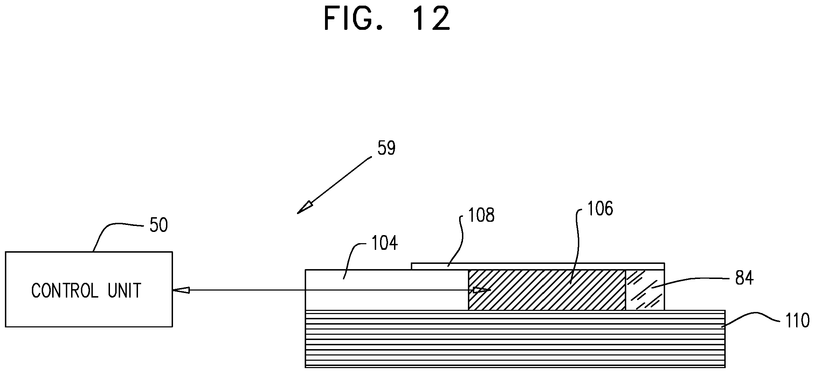

Fig. 12 is a schematic cross-sectional illustration of a sensing unit and optical system 59 like those described hereinabove, but comprising a non-cylindrical (e.g., planar) optical waveguide 104, in accordance with some applications of this invention. Non-cylindrical waveguide 104 conveys light to and from a cell and/or sensor protein chamber 106 optionally disposed on a substrate 110, and this light may also be reflected by one or more mirrors 84. The planar configuration of Fig. 12 may be used in combination with any of the configurations described herein with reference to Figs. 6A-11B or Figs. 13A-B.

Fig. 13A is a schematic cross-sectional illustration of another configuration of sensing unit 62, in accordance with some applications of the present invention. (Except for differences as described hereinbelow, the configuration shown in Fig. 13A is generally similar to that described hereinabove with reference to Fig. 6A.) In this configuration, sensing unit 62 comprises an internal cell chamber 120 and an external sensor protein chamber 122. External sensor protein chamber 122 is one implementation of sensor protein chamber 57, and internal cell chamber 120 is one implementation of cell chamber 55, both described hereinabove with reference to Fig. 5. Optionally, an internal membrane 60 separates internal cell chamber 120 from external sensor protein chamber 122. External membrane 58 surrounds external sensor protein chamber 122. Optionally, plug 78 closes the distal end of external membrane 58. Optical waveguide 48 is typically disposed parallel to a longitudinal axis 128 of external sensor protein chamber 122, and, optionally, alternatively or additionally, to a longitudinal axis 129 of internal cell chamber 120 (which is typically coaxial with external sensor protein chamber 122).

Typically, a first distal longitudinal segment 124 of external sensor protein chamber 122 surrounds (i.e., is disposed around) at least a portion of, e.g., at least at a proximal end portion of, such as all of, internal cell chamber 120, and a second proximal longitudinal segment 126 of external sensor protein chamber 122 does not surround (i.e., is not disposed around) internal cell chamber 120. Optical waveguide 48 is typically contiguous with second proximal longitudinal segment 126 of external sensor protein chamber 122. Typically, because internal cell chamber 120 occupies a portion of first distal longitudinal segment 124 of external sensor protein chamber 122, but not of second proximal longitudinal segment 126 of external sensor protein chamber 122, a cross-sectional area of external sensor protein chamber 122 is greater along second proximal longitudinal segment 126 than along first distal longitudinal segment 124. In addition, typically at least 60% of a volume of external sensor protein chamber 122 is disposed along second proximal longitudinal segment 126. As a result, the light transmitted by optical waveguide 48 interacts well with the sensor protein in external sensor protein chamber 122. For some applications, a diameter of optical waveguide 48 is equal to a diameter of sensing unit 62 and/or to a diameter of external sensor protein chamber 122.

Because first distal longitudinal segment 124 of external sensor protein chamber 122 surrounds at least a portion of, e.g., all of, internal cell chamber 120, a relatively large surface area is provided for allowing (a) transfer of analyte and nutrients between the subject's body and internal cell chamber 120, via external sensor protein chamber 122, and (b) transfer of the sensor protein from internal cell chamber 120 to external sensor protein chamber 122. In addition, because external sensor protein chamber 122 is typically disposed at the surface of sensing unit 62 along the entire sensing unit, a relatively large surface area is provided for allowing transfer of analyte and nutrients between the subject's body and external sensor protein chamber 122, via external membrane 58.

Fig. 13B is a schematic cross-sectional illustration of yet another configuration of sensing unit 62, in accordance with some applications of the present invention. (Except for differences as described hereinbelow, the configuration shown in Fig. 13B is generally similar to the configurations described hereinabove with reference to Figs. 6A and 13A.) In this configuration, sensing unit 62 comprises an external cell chamber 220 and an internal sensor protein chamber 222. Internal sensor protein chamber 222 is one implementation of sensor protein chamber 57, and external cell chamber 220 is one implementation of cell chamber 55, both described hereinabove with reference to Fig. 5. Optionally, an internal membrane 60 separates external cell chamber 220 from internal sensor protein chamber 222. External membrane 58 surrounds external cell chamber 220. Optionally, plug 78 closes the distal end of external membrane 58. Optical waveguide 48 is typically disposed parallel to a longitudinal axis 228 of internal sensor protein chamber 222, and, optionally, alternatively or additionally, to a longitudinal axis 229 of external cell chamber 220 (which is typically coaxial with internal sensor protein chamber 222).

Typically, a first proximal longitudinal segment 224 of external cell chamber 220 surrounds (i.e., is disposed around) at least a portion of, e.g., at least at a proximal end portion of, such as all of, internal sensor protein chamber 222, and a second distal longitudinal segment 226 of external cell chamber 220 does not surround (i.e., is not disposed around) internal sensor protein chamber 222. Optical waveguide 48 is typically contiguous with first proximal longitudinal segment 224 of internal sensor protein chamber 222. Typically, because internal sensor protein chamber 222 occupies a portion of first proximal longitudinal segment 224 of external cell chamber 220, but not of second distal longitudinal segment 226 of external cell chamber 220, a cross-sectional area of external cell chamber 220 is greater along second distal longitudinal segment 226 than along first proximal longitudinal segment 224. In addition, typically at least 60% of a volume of external cell chamber 220 is disposed along second distal longitudinal segment 226. For some applications, a diameter of optical waveguide 48 is equal to a diameter of internal sensor protein chamber 222.

Because first proximal longitudinal segment 224 of external cell chamber 220 surrounds at least a portion of, e.g., all of, internal sensor protein chamber 222, a relatively large surface area is provided for allowing transfer of the sensor protein from external cell chamber 220 to internal sensor protein chamber 222. In addition, because external cell chamber 220 is typically disposed at the surface of sensing unit 62 along the entire sensing unit, a relatively large surface area is provided for allowing transfer of analyte and nutrients between the subject's body and external cell chamber 220, via external membrane 58.

Reference is made to both Figs. 13A and 13B. Optionally, internal membrane 60 and external membrane 58 have the different MWCO described hereinabove with reference to Fig. 5.

Reference is still made to both Figs. 13A and 13B. The configuration of sensing unit 62 described with reference to Fig. 13A may provide a faster response time due to the single membrane through which the analyte (e.g., glucose) must pass before being detected. In addition, the configuration of sensing unit 62 described with reference to Fig. 13A may allow an easier assembly process since the cells may be encapsulated in internal cell chamber 120 prior to the assembly of the full sensing unit 62; in addition, the encapsulated cells may be tested before internal cell chamber 120 is placed in external sensor protein chamber 122. On the other hand, the configuration of sensing unit 62 described with reference to Fig. 13B may allow the sensing unit 62 to contain a higher volume of cells, which may generate a higher biosensor protein density and proximity of the cells to the nutrients diffusing from the outside of the sensing unit.

Reference is still made to both Figs. 13A and 13B. For some applications, sensing unit 62 further comprises a mechanical support 130 positioned radially between internal and external membranes 60 and 58, which may provide mechanical stability to the sensing unit. The mechanical support may comprise a metal pipe perforated, e.g., using laser cutting, providing windows for diffusion of molecules while maintaining the overall rigidity of the metal pipe. Mechanical support 130 may also be provided in the other configurations of sensing unit 62 described hereinabove with reference to Figs. 6A-12.

In order to enable immediate testing and qualification of sensing unit 62, and make efficient use of device shelf life, there are benefits to manufacturing sensing unit 62 as close as reasonably possible to its typical operational state and to eliminate as much as possible any "maturation" gradients. In the case of a device such as sensing unit 62 that is based on secreted biosensor protein, the long-term steady state concentration of sensor protein in sensor protein chamber 57 is determined by the balance between (a) protein generation rate by the cells, and (b) protein loss because of catabolism of the proteins, caused by proteases and other factors and possibly protein leakage out of the device. This steady state may take a week or more to reach, thus preventing immediate testing of the device after manufacture and the need for a maturation period.

In some applications of the present invention, the device manufacturing process comprises the loading of purified biosensor protein, at the expected steady state concentration, into sensor protein chamber 57. The protein may be separately manufactured in the same cells, in other cells, or in bacteria and purified from the growth medium. An additional benefit of providing a steady-state level of sensor protein in the manufacturing process is that the number of cells required for the operation of the sensing unit is only the number necessary to support the steady state over the long term, thereby allowing a smaller number of cells and thus a smaller volume for cell chamber 55.

Reference made to Figs. 5-13B. For some applications, during assembly of sensing unit 62, either (a) cell chamber 55 and sensor protein chamber 57 or (b) sensor protein chamber 57 is pre-loaded with biosensor protein purified from a cell culture produced *in vitro.* Such an assembly process provides immediate functionality to the sensing unit, without the need to wait for biosensor protein density to accumulate as a result of secretion from the cells. A more stable biosensor density in the sensing unit immediately upon implantation also may produce higher accuracy during the initial period following implantation and longer calibration intervals.

For any of the configurations of cell chamber 55 described hereinabove with reference to Figs. 6B, 7B, and 13A, the configurations of scaffold material 28 described hereinabove with reference to Figs. 1-3B may be used to contain the cells. Typically, cell chamber 55 comprises only the portion of scaffold 28 to which cells are attached, and not the portion for coupling with optical waveguide 48. In addition, when used in these configurations, scaffold material 28 is not necessarily optically transparent.

High concentrations of the sensor protein may enhance the intensity of the optical signal. For some applications of the present invention, in order to achieve a high local concentration of sensor protein, the sensor protein is targeted to specific surfaces of the apparatus which enjoy a higher collection efficiency by the optics. Targeting may be achieved, for example, by creating a specific interaction between the protein and the surface, e.g., by the addition of a linker to the protein. The linker has enhanced binding to the specific surface either through a physical interaction (e.g., a hydrophobic or hydrophilic interaction) or through a specific biological interaction (e.g., a biotin-avidin interaction).

Reference is made to Figs. 1-13B. In many configuration of the sensing units described herein, the effective optical length in the transparent parts of the sensing unit (e.g., the cell chamber or protein chamber) is a few millimeters, based on the typical absorption of the optical signal. However, for several reasons there is a benefit to minimizing the length of the measured volume, including that the collection efficiency is better closer to the optical fiber, and that the overall dimensions of the device are smaller. Therefore, in any of the configurations described herein, a mirror may optionally be provided at the end of the transparent region, e.g., as described hereinabove with reference to Figs. 10B, 11A, and/or 12. One aspect of the design of the devices described herein incorporates improving the optical coupling.

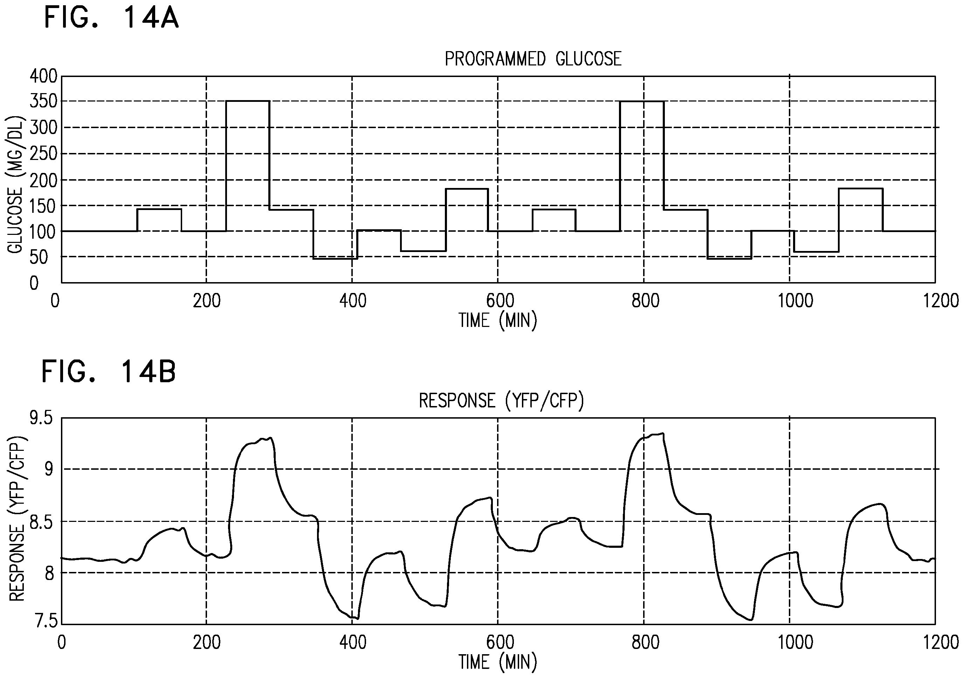

Reference is now made to Figs. 14A-B, which are graphs showing the measurement of glucose in accordance with an experiment conducted by the inventors. This *in vitro* experiment tested a device comprising a sensing unit similar sensing unit 62 in the configuration described hereinabove with reference to Fig. 13B. The sensing unit was placed in a computer-controlled perfusion system, including closed-loop circulation of a growth medium and temperature control, and the concentration of glucose in the circulating growth medium was controlled to have the values over time shown in Fig. 14A. An optical waveguide similar to optical waveguide 48 transmitted light to the sensing unit, and fluorescent light emitted by the sensor protein in the sensing unit was detected. The emission levels of the respective wavelengths emitted by yellow fluorescent protein (YFP) and by cyan fluorescent protein (CFP) were measured. The ratio of the YFP to CFP emission levels was calculated over time, as shown in Fig. 14B.

As can be seen in the graphs of Figs. 14A-B, there was a strong correlation between the controlled glucose level in the medium and the ratio of YFP to CFP. These data indicate that the sensing units described herein can accurately detect glucose levels.

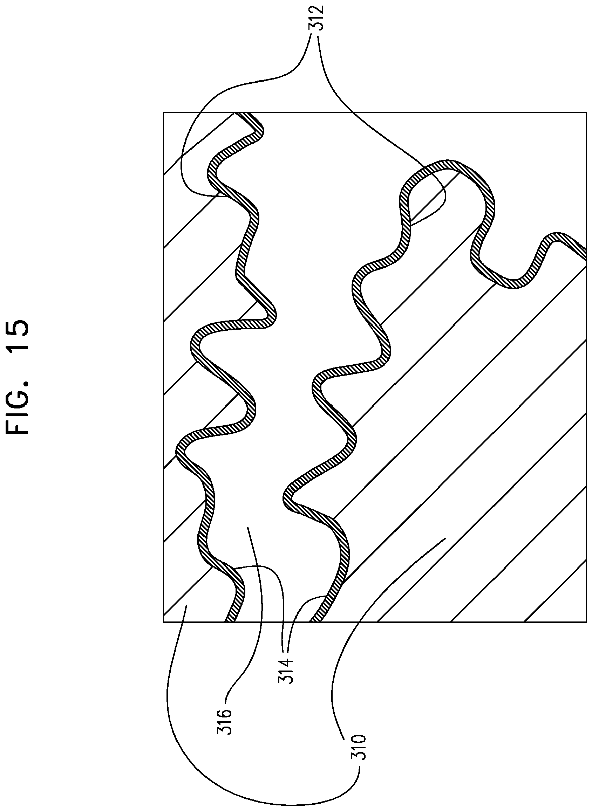

Reference is now made to Figs. 15-19. One of the challenges in the design of a cell-based implantable device is the maintenance of a significant population of cells over the long term, e.g., over a year or longer. In accordance with some applications of the present invention, techniques are provided for maintaining a desired cell population size over time, including both:
- restraining cell population growth, i.e., cell proliferation, in order to avoid over-population that leads to a shortage of nutrients in cells farther from the edge of the device, which would create a necrotic core of cells that eventually intoxicates the entire cell population; and
- allowing limited cell proliferation to replace cells that die over time, in order to prevent dwindling of the cell population in the device, which would eventually render the device dysfunctional.

Reference is made to Fig. 15, which is a schematic cross-sectional diagram of a three-layer cell encapsulation structure 300, in accordance with an application of the present invention. In order to balance the above-mentioned conflicting goals and preserve a generally constant cell population over a long period of time, e.g., at least one year, encapsulation structure 300 comprises a substantially non-degradable three-dimensional scaffold 310 having surfaces 312 to which cells 314 are attached, and a hydrogel 316, which is applied to cells 314. Hydrogel 316 is typically inert, and may comprise, for example, alginate, a PEG hydrogel, or another biocompatible hydrogel.

Scaffold 310, cells 314, and hydrogel 316 are arranged such that cells 314 are sandwiched in spaces between hydrogel 316 and surfaces 312 of scaffold 310. Cells 314 are arranged in monolayers on at least 50%, such as at least 70%, e.g., at least 90% (for example, 100%) of an aggregate surface area of surfaces 312 of scaffold 310 (the "aggregate surface area" is the sum of the surface areas of all of the surfaces of the scaffold). This arrangement allows mobility and proliferation of cells 314 in the spaces between hydrogel 316 and surfaces 312 of scaffold 310, and prevents the mobility and the proliferation of cells 314 to locations outside of the spaces between hydrogel 316 and surfaces 312 of scaffold 310. Typically, cells 314 occupy at least 75% of the aggregate volume of the spaces between hydrogel 316 and surfaces 312 of scaffold 310. Cells within the spaces between hydrogel 316 and surfaces 312 of scaffold 310 that die, such as because of stress or apoptosis, leave a space upon disintegration. The structure provided by the surface of the scaffold on one side and the hydrogel on the other side maintain the patency of this space until one or more neighboring cells proliferate into the space.

Thus, in any local microscopic environment encapsulation structure 300 comprises a three-layer stack of (a) surface 312 of solid scaffold 310, (b) cells 314, and (c) hydrogel 316, in this order. The cells at any location are thus generally limited to a monolayer, allowing free mobility and proliferation of the cells within the narrow space between the scaffold and the hydrogel, but preventing any proliferation into the rest of the volume and creation of three-dimensional cell structures.

Scaffold 310 provides a three-dimensional structure with a high aggregate surface area, and high surface-to-volume ratio, which makes efficient use of the three-dimensional volume of the chamber. The surfaces of the scaffold, although often not flat, serve effectively as a two-dimensional substrate for seeding, growth, and attachment of the cells. If hydrogel 316 were not provided over the monolayer of the cells, the cells typically grow in three dimensions, away from the surfaces to which they are attached. Such three-dimensional growth would generally result in undesirable over-population, as described above. In addition, for many cell types, cell viability and protein expression, including expression of the sensor protein, are significantly enhanced when cells are attached and spread. Thus cells in this configuration will survive longer and function better than dispersed cell, e.g., cells dispersed in a hydrogel scaffold.

For some applications, encapsulation structure 300 further comprises a chamber, such as sensor protein chamber 57 in any of the configurations described hereinabove with reference to Figs. 5-13B. The scaffold, the cells, and the hydrogel are contained in the chamber. For some applications, encapsulation structure 300 further comprises an external membrane, such as external membrane 58, which surrounds at least a portion of the chamber, such as the entire chamber.

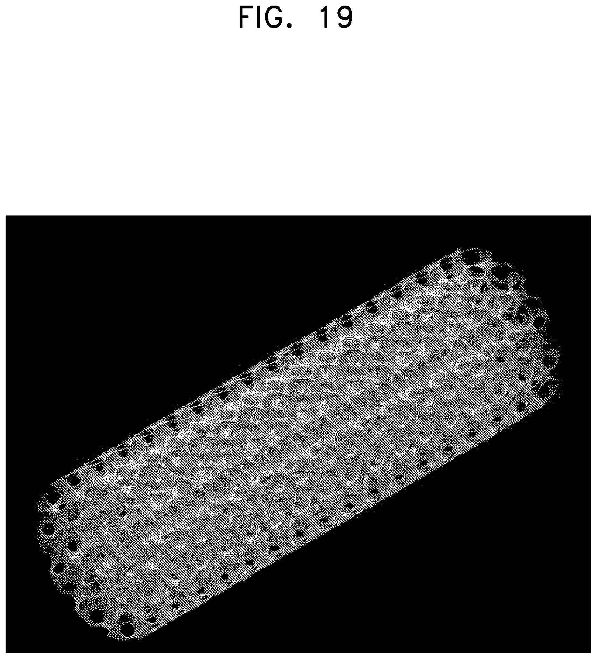

For some applications, scaffold 310 comprises:
- microcarrier beads, configured to allow cell attachment on the surfaces of the beads (e.g., Cytodex® surface microcarriers (GE Healthcare Bio-Sciences, Sweden)) (as used in the present application, including in the claims, the elements of scaffold 310 are not necessarily structurally connected to one another, but collectively provide a solid support structure for growth and attachment of cells);
- fibers, either in an ordered form such as a braid or in a chaotic and/or random form, e.g., created by electro-spinning;
- a rigid structure, such as scaffold material 28, described hereinabove with reference to Figs. 1-3B. The rigid structure may be fabricated, for example, by 3D printing (for example, using MED610 or plastic molding. If the configurations described with reference to Figs. 1-3B are used, scaffold 310 typically includes only the portion of scaffold material 28 to which cells are attached, and not the portion for coupling with optical waveguide 48; in addition, scaffold 310, unlike scaffold material 28, is not necessarily optically transparent. Scaffold 310 may be shaped so as to define a plurality of wells 30, as described hereinabove, and/or may implement any of the other features of scaffold material 28; or
- a sponge structure having a plurality of interconnected internal pores, such as described hereinbelow with reference to Fig. 19. The sponge structure may be fabricated, for example, by 3D printing using biocompatible materials, such as MED610. Typically, the pore size is at least 50 um in diameter, such as at least 100 um in diameter.

In accordance with an application of the present invention, encapsulation structure 300 is manufactured by the following process:
- providing a substantially non-degradable three-dimensional scaffold 310 having surfaces 312 suitable for cell attachment and growth. Optionally surface 312 are treated for enhancement of cell adhesion, e.g., by coating with ECM proteins, e.g., collagen, fibrinogen, or by plasma treatment, to provide surface charge;
- seeding cells 314 on surfaces 312 and allowing cell proliferation to reach at least 70% confluence, such as at least 90%, e.g., 100% confluence; and
- before cells 314 form three-dimensional structures on 50% (typically 30%, such as 10%, e.g., any) of an aggregate surface area of surfaces 312, filling, with hydrogel 316, a volume of encapsulation structure 300 which is not already occupied by cells 314 or scaffold 310, thereby preventing additional cell proliferation into the volume of encapsulation structure 300 which is not already occupied by the cells or the scaffold.

Ideally, hydrogel 316 penetrates all spaces in encapsulation structure 300 that are not occupied by scaffold 310 or cells 314. Therefore, the minimum feature size of surfaces 312 is typically at least a few tens of micrometers.

Encapsulation structure 300 may combine at least three benefits: (a) good cell attachment, leading to better cell viability and expression, lacking in simpler systems that for example use hydrogel as a scaffold, (b) prevention of over-population which often leads to a necrotic core, because of a limited number of cells and open diffusion channels to the cells via the hydrogel, and (c) enablement of cell mobility and proliferation within a two-dimensional culture, thereby enabling long-term steady state population.

For some applications, cells 314 are differentiated cells, such as terminally-differentiated cells. For other applications, cells 314 are stem cells. For some applications, cells 314 are genetically engineered to produce a fluorescent sensor protein having a binding site for an analyte, such as glucose, the fluorescent sensor protein being configured to emit fluorescent light in response to excitation light, such as using the techniques described hereinabove.

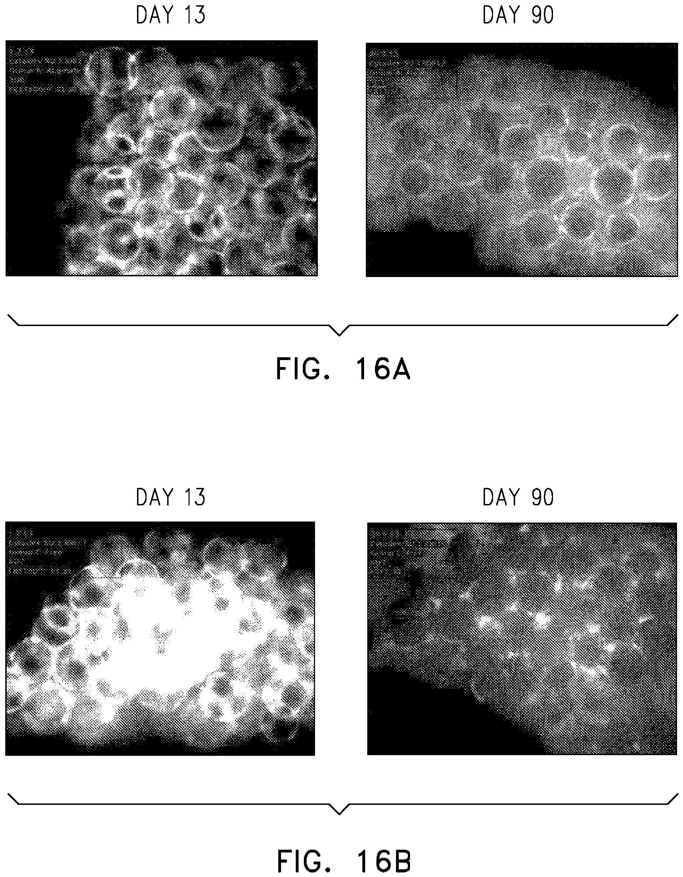

Reference is now made to Figs. 16A-18, which show results of an experiment conducted by the inventors. The inventors manufactured an encapsulation structure similar to that described hereinabove with reference to Fig. 15, using Cytodex® 1 surface microcarrier beads (200-220 um) as scaffold 310. The beads were seeded with cells which were previously stably transfected with a gene for the fluorescent protein similar to that described in above-mentioned PCT Patent Application Publication WO 2006/006166. Upon reaching a high level of cell coverage (greater than 80%), the beads where densely filled into hollow fiber membranes. In the experimental configuration the beads where mixed with alginate prior to injection into the hollow fiber membranes and the alginate was cross-linked after injection, to form hydrogel 316, as described above. In the other control configuration, the beads were injected into the hollow fiber membranes with growth medium, with the spaces between the beads left empty of hydrogel. Both configurations were then incubated in a growth medium for up to 90 days.

The resulting dynamics of cell populations are shown in Figs. 16A-B. As can be seen in Fig. 16A, in the first experimental configuration, the cells maintained a significant, protein-expressing population strictly attached to the bead surfaces, whereas in the second control configuration, the cells formed three-dimensional structures primarily in the spaces between the beads, as can be seen in Figs. 16B. In addition, while the cells of the control configuration enjoyed a quick period of growth, starting from a similar cell population on day 1 (not shown), the cells of the control configuration experienced fast decay in overall protein expression following day 13, while the cells of the experimental configuration achieved nearly a steady state.

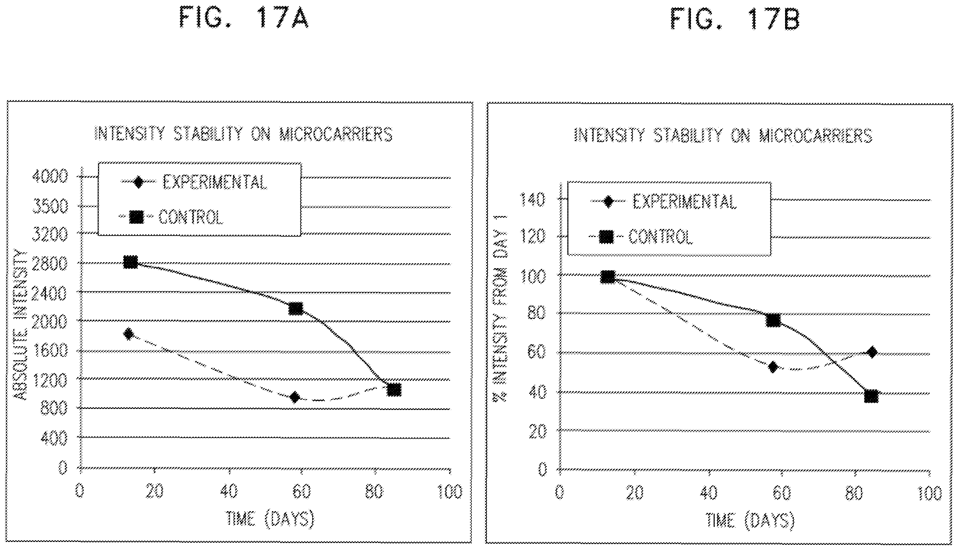

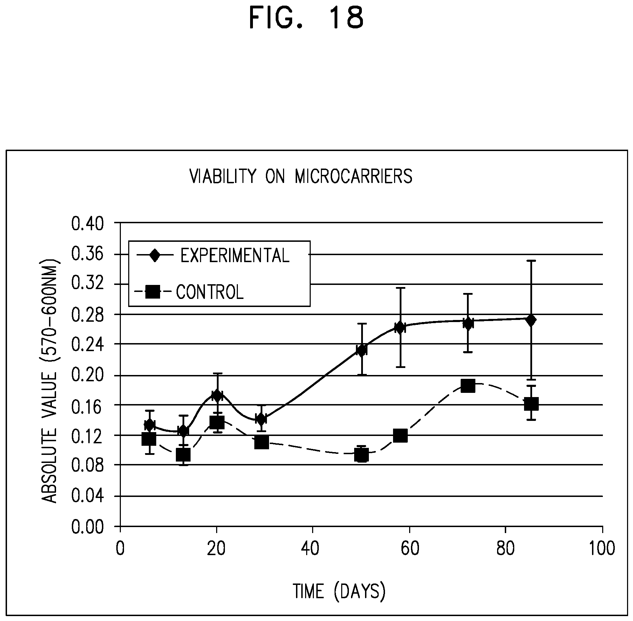

Figs. 17A-B shows the results of an intensity analysis, and Fig. 18 shows PrestoBlue metabolism tests performed in the experiment. These graphs demonstrate that experimental beads-in-alginate configuration supported steady-state viability and protein expression. It can thus be maintained that for even longer terms than 90 days the benefit of the proposed system, as implemented here in the first configuration, are very significant, enabling the performance of an implantable cell-based device over periods of many months.

Reference is made to Fig. 19, which is a photograph of an artificial sponge structure, in accordance with an application of the present invention. As mentioned above, the sponge structure may serve as scaffold 310. For some applications, the sponge structure is fabricated by 3D printing. By way of example, the shown sponge structure is shaped so as to define interconnected internal pores having sizes of between 80 and 120 um in diameter. The shown sponge structure has a high surface-to-volume ratio.

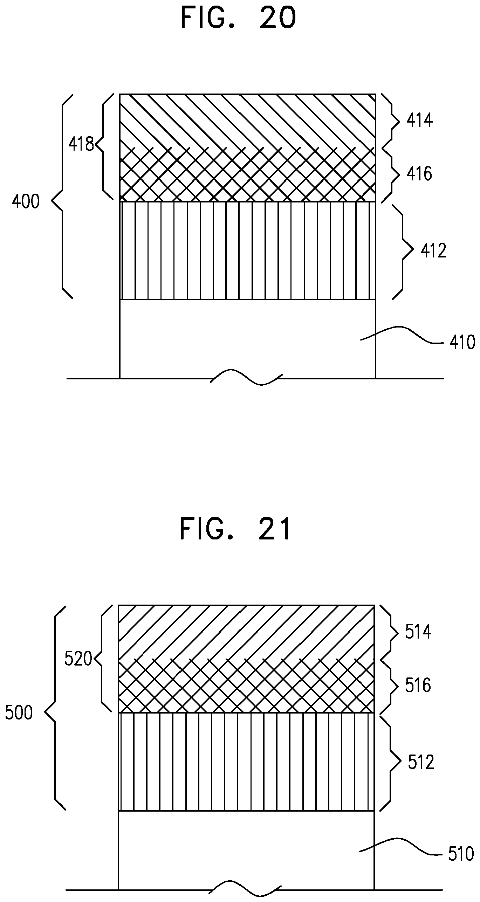

Reference is now made to Fig. 20, which is a schematic cross-sectional illustration of a multi-layer immunoisolation system 400, in accordance with an application of the present invention. The viability of cells within a cell-based device strongly depends on an ample supply of oxygen. Generally, the foreign body response following device implantation creates dense fibrotic tissue that encapsulates the device, substantially reducing oxygen diffusion to the device from the blood circulation. Therefore, the viability of cells inside a cell-based device is enhanced by substantial vascularization of the tissue as close as possible to the implanted device, which increases oxygen levels at the device surface. More specifically, for a glucose measurement device, the creation of a dense fibrotic tissue is a potential diffusion barrier for glucose, leading to a time delay between glucose levels in the tissue and glucose levels measured by the device. Such dense fibrotic tissue should thus be avoided in order to maintain the accuracy of the glucose measurement.

Multi-layer immunoisolation system 400 is configured to enhance long-term function of an implantable cell-based device 410. Multi-layer immunoisolation system 400 is disposed at an external surface of device 410. For example, multi-layer immunoisolation system 400 may be integrated into any of the sensing devices described herein instead of, or as an implementation of, external membrane 58.

Multi-layer immunoisolation system 400 comprises at least the following three layers:
- a lower (inner) membrane layer 412, which is disposed at an external surface of device 410 (lower membrane layer 412 either is shaped so as to define the external surface of device 410, or is fixed to the external surface of device 410);
- an upper (outer) neovascularization layer 414; and
- a middle protective layer 416, disposed between lower membrane layer 412 and upper neovascularization layer 414.

Multi-layer immunoisolation system 400 comprises a biodegradable scaffold 418. Before biodegrading, biodegradable scaffold 418 spans both upper neovascularization layer 414 and middle protective layer 416, such that upper neovascularization layer 414 comprises a first upper portion of biodegradable scaffold 418, and middle protective layer 416 comprises a second lower portion of biodegradable scaffold 418.

In addition, middle protective layer 416 further comprises a non-biodegradable hydrogel that impregnates the second lower portion of biodegradable scaffold 418. Upper neovascularization layer 414, which comprises the first upper portion of biodegradable scaffold 418, is not impregnated with the hydrogel.

Biodegradable scaffold 418 serves at least two functions:
- during implantation of device 410, biodegradable scaffold 418 protects the soft hydrogel of middle protective layer 416 from strong shear forces which might otherwise pull off the soft hydrogel; and
- after implantation of device 410, biodegradable scaffold 418 promotes vascularization of the tissue that grows into upper neovascularization layer 414 (but not into middle protective layer 416), until biodegradable scaffold 418 eventually degrades and is totally absorbed.

Upon biodegradation of biodegradable scaffold 418, middle protective layer 416 (now comprising primarily the hydrogel) remains attached to lower membrane layer 412. Middle protective layer 416 typically serves to (a) prevent attachment of proteins to lower membrane layer 412, thereby minimizing the creation of a fibrotic tissue, and/or (b) repel large proteins, thereby minimizing the fouling of lower membrane layer 412. The high water content of the hydrogel of middle protective layer 416 prevents the attachment of various proteins, so that immune system cells are less likely to attach to the tissue-hydrogel interface, thereby minimizing the overall immune response. Typically, the hydrogel of middle protective layer 416 has a thickness of at least 50 um, e.g., at least 100 um, such as in order to enable reactive oxygen species (ROS) decay between inflamed tissue and the device cells. Without the use of the techniques described herein, it is generally difficult to attach a hydrogel to a membrane, particularly with a thickness of more than a few um.

As a result of this triple-layer protection, the tissue surrounding device 410 is characterized by high vascularization and minimal fibrosis.

Typically, lower membrane layer 412 (and lower membrane 512, described hereinbelow with reference to Fig. 21) has a MWCO of at least 5 KDa, no more than 50 KDa, and/or between 5 and 50 KDa. (Typically, the MWCO of a membrane should be no more than one-third of the size of the molecule to be blocked. Thus, for blocking IgG, which generally has a size of about 150 KDa, a membrane of 50Ka MWCO or lower should be used.) For some applications, lower membrane layer 412 comprises polysulfone (PS), polyethersulfone (PES), modified polyethersulfone (mPES), or polytetrafluoroethylene (PTFE, Teflon®).

Typically, biodegradable scaffold 418 is highly porous, and has an average pore size of at least 5 um, no more than 50 um, and/or or between 5 and 50 um. For some applications, the scaffold comprises a mesh. Biodegradable scaffold 418 may comprise a polymer, such as polylactic acid (PLA), poly(DL-lactic-co-glycolic acid) (PLGA), poly(3-hydroxypropionic acid) (P(3-HP)), or 3-hydroxypropionic acid (3-HP). Biodegradable polymers and the products of their degradation are typically non-toxic, so as to not evoke a strong immune response. Additionally, biodegradable polymers typically maintain good mechanical integrity until degraded in order to evoke enhanced vascularization in its vicinity. Finally, biodegradable polymers typically have controlled degradation rates leading to complete disintegration in the body within a few weeks to a few months, which is enough time to evoke vascularization but not become a potential annoyance for the patient a long time after the device is explanted.

Biodegradable scaffold 418 (of upper neovascularization layer 414 and the middle protective layer 416 in combination) typically has a thickness of between 100 and 300 um and promotes neovascularization by virtue of the large pore size and the slow biodegradation effect. As mentioned above, the scaffold additionally holds the hydrogel layer in place. For some applications, biodegradable scaffold 418 is fixed to the upper (outer) surface of membrane layer 412 by gluing. Alternatively or additionally, for some applications, biodegradable scaffold 418 is fixed to the upper (outer) surface of membrane layer 412 by being directly deposited using electro spinning, i.e., the scaffold is electro spun onto the membrane.

The hydrogel (and hydrogel 520, described hereinbelow with reference to Fig. 21) may comprise Poly(ethylene glycol) (PEG), a zwitterionic hydrogel, or any other non-biodegradable hydrogel. The hydrogel is typically impregnated into the second lower portion of biodegradable scaffold 418 in liquid form, and then cross-linked. Applying the hydrogel only to the second lower portion, but not the first upper portion, of biodegradable scaffold 418 may be performed, for example, by (a) impregnating the entire thickness of the biodegradable scaffold, and then drying the hydrogel from the first upper portion, e.g., by soaking the hydrogel into a dry absorbing material, or by a combination of high temperatures and low pressure, or (b) soaking the entire thickness of the biodegradable scaffold with the liquid hydrogel (without a cross-linker), and injecting the cross-linker through lower membrane layer 412, e.g., during application of UV radiation, resulting in preferential crosslinking of the hydrogel from the bottom up; this crosslinking process is halted before the hydrogel in the first upper portion of the biodegradable scaffold is cross-linked, and the remaining hydrogel is washed out of the scaffold.

Reference is now made to Fig. 21, which is a schematic cross-sectional illustration of a multi-layer immunoisolation system 500, in accordance with an application of the present invention. Other than as described below, multi-layer immunoisolation system 500 may have any of the characteristics and properties of multi-layer immunoisolation system 400, described hereinabove with reference to Fig. 20.

Multi-layer immunoisolation system 500 is configured to enhance long-term function of an implanted cell-based device 510. Multi-layer immunoisolation system 500 is disposed at an external surface of device 510. For example, multi-layer immunoisolation system 500 may be integrated into any of the sensing devices described herein instead of, or as an implementation of, external membrane 58.

Multi-layer immunoisolation system 500 comprises at least the following three layers:
- a lower (inner) membrane layer 512, which is disposed at an external surface of device 510 (lower membrane layer 512 either is shaped so as to define the external surface of device 510, or is fixed to the external surface of device 510);
- an upper (outer) protective layer 514; and
- a middle attachment layer 516, which is disposed between lower membrane layer 512 and upper protective layer 514, and which tightly fixes upper protective layer 514 to lower membrane layer 512

Middle attachment layer 516 comprises a non-biodegradable scaffold, which is tightly fixed to lower membrane layer 512, such as by being deposited directly on the membrane using electro spinning, i.e., the scaffold is electrospun onto the membrane. Typically, the scaffold is highly porous, and may comprise, for example, a polymer such as polyurethane, polyvinylidene fluoride (PVDF), or polyethylene terephthalate (PET). Middle attachment layer 516 typically has a thickness of between 50 and 100 um.

Multi-layer immunoisolation system 500 comprises a non-biodegradable hydrogel 520, which spans both upper protective layer 514 and middle attachment layer 516. In other words, middle attachment layer 516 comprises a first portion of hydrogel 520, and upper protective layer 514 comprise a second portion of hydrogel 520. Hydrogel 520 is impregnated in the scaffold of middle attachment layer 516, and extends above the scaffold, i.e., in a direction away from lower membrane 516, so as to provide upper protective layer 514. Upper protective layer 514 does not comprise the scaffold. As a result, the scaffold is not exposed to tissue, thereby reducing the likelihood that multi-layer immunoisolation system 500 generates an immune response.

Middle attachment layer 516 holds the hydrogel of upper protective layer 514 in place on lower membrane layer 512. Upper protective layer 514 has a smooth upper (outer) surface, which results in low biofouling of lower membrane layer 512, allowing the membrane to efficiently diffuse nutrients into device 510 even after a long implantation period. In addition, upper protective layer 514 protects device 510 by presenting a highly biocompatible surface to the tissue. Upper protective layer 514 typically has a thickness of between 50 and 200 um.

It will be appreciated by persons skilled in the art that the present invention is not limited to what has been particularly shown and described hereinabove. Rather, the scope of the present invention is defined by the claims.

## Claims

1. An implantable encapsulation structure (300) containing cells for implantation into a human subject, the implantable encapsulation structure (300) comprising: a substantially non-degradable three-dimensional scaffold (310) having surfaces (312), a hydrogel (316), cells (314), wherein the cells (314) are arranged in monolayers on at least 50% of an aggregate surface area of the surfaces (312) of the scaffold (310), and the cells (314) are attached to the surfaces (312) of the scaffold (310), and the hydrogel (316) is attached to the cells (314), **characterized in that** the scaffold (310), the cells (314) and the hydrogel (316) are arranged such that the cells (314) are sandwiched in spaces between the hydrogel (316) and the surfaces (312) of the scaffold (310), thereby allowing mobility and proliferation of the cells (314) in the spaces between the hydrogel (316) and the surfaces (312) of the scaffold (310), and preventing the mobility and the proliferation of the cells (314) to locations outside of the spaces between the hydrogel (316) and the surfaces (312) of the scaffold (310).

2. The implantable encapsulation structure (300) according to claim 1, wherein the cells (314) are arranged in the monlayers on at least 70% of the aggregate surface area of the surfaces (312) of the scaffold (310).

3. The implantable encapsulation structure (300) according to claim 1, wherein the cells (314) are arranged in the monolayers on at least 90% of the aggregate surface area of the surfaces (312) of the scaffold (310).

4. The implantable encapsulation structure (300) according to claim 1, further comprising:
a chamber (57) in which the scaffold (310), the cells (314) and the hydrogel (316) are contained.

5. The implantable encapsulation structure (300) according to claim 4, further comprising:
an external membrane (58) which surrounds at least a portion of the chamber (57).

6. The implantable encapsulation structure (300) according to claim 1, wherein the cells (314) are differentiated cells, which are attached to the surfaces (312) of the scaffold (310).

7. The implantable encapsulation structure (300) according to claim 6, wherein the differentiated cells are terminally-differentiated cells, which are attached to the surfaces (312) of the scaffold (310).

8. The implantable encapsulation structure (300) according to claim 1, wherein the cells (314) are stem cells, which are attached to the surfaces (312) of the scaffold (310).

9. The implantable encapsulation structure (300) according to claim 1, wherein the cells (314) are genetically engineered to produce a fluorescent sensor protein having a binding site for an analyte, the fluorescent sensor protein being configured to emit fluorescent light in response to excitation light, optionally the analyte is glucose.

10. The implantable encapsulation structure (300) according to any of claims 1 to 9, wherein the scaffold (310) comprises microcarrier beads, optionally filled into hollow fiber membranes.

11. The implantable encapsulation structure (300) according to any of claims 1 to 9, wherein the scaffold (310) comprises fibers, optionally in braid form or in chaotic or random form.

12. The implantable encapsulation structure (300) according to any of claims 1 to 9, wherein the scaffold (310) comprises a sponge structure having a plurality of interconnected internal pores, optionally greater than 50 µm in diameter, optionally greater than 100 µm in diameter.

13. The implantable encapsulation structure (300) according to any of claims 1 to 9, wherein the scaffold (310) is a rigid structure (28).

14. The implantable encapsulation structure (300) according to claim 13, wherein the rigid structure (28) is shaped so as to define a plurality of wells (30).

15. A method of manufacturing an implantable encapsulation structure (300) according to claim 1 containing cells for implantation into a human subject, comprising: providing a substantially non-degradable three-dimensional scaffold (310) having surfaces (312) for cell attachment and growth; seeding cells (314) on the surfaces (312) of the scaffold (310) and allowing cell proliferation to reach at least 70% confluence, **characterized in that** before the cells (314) form three-dimensional structures on 50% of an aggregate surface area of the surfaces (312) of the scaffold (310), filling, with a hydrogel (316), a volume of the scaffold (310) which is not already occupied by the cells (314), thereby preventing additional proliferation of the cells (314), whereby the cells (314) are arranged in monolayers on at least 50% of an aggregate surface area of the surfaces (312) of the scaffold (310).

## Patentansprüche

1. Implantierbare Einkapselungsstruktur (300), die Zellen zum Implantieren in ein menschliches Subjekt beinhaltet, wobei die implantierbare Einkapselungsstruktur (300) Folgendes umfasst: ein im Wesentlichen nicht abbaubares dreidimensionales Gerüst (310), das Oberflächen (312), ein Hydrogel (316) und Zellen (314) aufweist, wobei die Zellen (314) in Monoschichten auf mindestens 50 % eines Gesamt-Oberflächenbereichs der Oberflächen (312) des Gerüsts (310) angeordnet sind, und die Zellen (314) an den Oberflächen (312) des Gerüsts (310) befestigt sind, und das Hydrogel (316) an den Zellen (314) befestigt ist, **dadurch gekennzeichnet, dass** das Gerüst (310), die Zellen (314) und das Hydrogel (316) so angeordnet sind, dass die Zellen (314) in Räumen zwischen dem Hydrogel (316) und den Oberflächen (312) des Gerüsts (310) eingebettet sind, wodurch Mobilität und Proliferation der Zellen (314) in die Räume zwischen dem Hydrogel (316) und den Oberflächen (312) des Gerüsts (310) ermöglicht wird, und die Mobilität und Proliferation der Zellen (314) zu Stellen außerhalb der Räume zwischen dem Hydrogel (316) und den Oberflächen (312) des Gerüsts (310) verhindert wird.

2. Implantierbare Einkapselungsstruktur (300) nach Anspruch 1, wobei die Zellen (314) in den Monoschichten auf mindestens 70 % des Gesamt-Oberflächenbereichs der Oberflächen (312) des Gerüsts (310) angeordnet sind.

3. Implantierbare Einkapselungsstruktur (300) nach Anspruch 1, wobei die Zellen (314) in den Monoschichten auf mindestens 90 % des Gesamt-Oberflächenbereichs der Oberflächen (312) des Gerüsts (310) angeordnet sind.

4. Implantierbare Einkapselungsstruktur (300) nach Anspruch 1, die ferner Folgendes umfasst:
eine Kammer (57), in der das Gerüst (310), die Zellen (314) und das Hydrogel (316) beinhaltet sind.

5. Implantierbare Einkapselungsstruktur (300) nach Anspruch 4, die ferner Folgendes umfasst:
eine Außenmembran (58), die mindestens einen Abschnitt der Kammer (57) umgibt.

6. Implantierbare Einkapselungsstruktur (300) nach Anspruch 1, wobei die Zellen (314) differenzierte Zellen sind, die an den Oberflächen (312) des Gerüsts (310) befestigt sind.

7. Implantierbare Einkapselungsstruktur (300) nach Anspruch 6, wobei die differenzierten Zellen terminal differenzierte Zellen sind, die an den Oberflächen (312) des Gerüsts (310) befestigt sind.

8. Implantierbare Einkapselungsstruktur (300) nach Anspruch 1, wobei die Zellen (314) Stammzellen sind, die an den Oberflächen (312) des Gerüsts (310) befestigt sind.

9. Implantierbare Einkapselungsstruktur (300) nach Anspruch 1, wobei die Zellen (314) genetisch verändert wurden, um ein Fluoreszenzsensorprotein zu produzieren, das eine Bindungsstelle für einen Analyten aufweist, wobei das Fluoreszenzsensorprotein konfiguriert ist, um Fluoreszenzlicht als Reaktion auf Anregungslicht zu emittieren, wobei optional das Analyt Glukose ist.

10. Implantierbare Einkapselungsstruktur (300) nach einem der Ansprüche 1 bis 9, wobei das Gerüst (310) Mikroträgerkügelchen umfasst, die optional in Hohlfasermembranen gefüllt werden.

11. Implantierbare Einkapselungsstruktur (300) nach einem der Ansprüche 1 bis 9, wobei das Gerüst (310) Fasern umfasst, optional in Zopfform oder in chaotischer oder zufälliger Form.

12. Implantierbare Einkapselungsstruktur (300) nach einem der Ansprüche 1 bis 9, wobei das Gerüst (310) eine Schwammstruktur umfasst, die eine Vielzahl von miteinander verbundenen inneren Poren aufweist, die optional größer als 50 µm im Durchmesser sind, optional größer als 100 µm im Durchmesser.

13. Implantierbare Einkapselungsstruktur (300) nach einem der Ansprüche 1 bis 9, wobei das Gerüst (310) eine starre Struktur (28) ist.

14. Implantierbare Einkapselungsstruktur (300) nach Anspruch 13, wobei die starre Struktur (28) so geformt ist, dass sie eine Vielzahl von Vertiefungen (30) definiert.

15. Verfahren zum Herstellen einer implantierbaren Einkapselungsstruktur (300) nach Anspruch 1, die Zellen zum Implantieren in ein menschliches Subjekt beinhaltet, die Folgendes umfasst: Bereitstellen eines im Wesentlichen nicht abbaubaren dreidimensionalen Gerüsts (310), das Oberflächen (312) für die Zellbefestigung und das Zellwachstum aufweist; Säen von Zellen (314) auf den Oberflächen (312) des Gerüsts (310) und Ermöglichen, dass die Zellproliferation eine Konfluenz von mindestens 70 % erreicht, **dadurch gekennzeichnet, dass**, bevor die Zellen (314) dreidimensionale Strukturen auf 50 % eines Gesamt-Oberflächenbereichs der Oberflächen (312) des Gerüsts (310) bilden, ein Volumen des Gerüsts (310), das nicht bereits von den Zellen (314) besetzt ist, mit einem Hydrogel (316) gefüllt wird, damit zusätzliche Proliferation der Zellen (314) verhindert wird, wodurch die Zellen (314) in Monoschichten auf mindestens 50 % eines Gesamt-Oberflächenbereichs der Oberflächen (312) des Gerüsts (310) angeordnet werden.

## Revendications

1. Structure d'encapsulation pouvant être implantée (300) contenant des cellules, destinée à une implantation chez un sujet humain, la structure d'encapsulation pouvant être implantée (300) comprenant : un échafaudage tridimensionnel sensiblement non dégradable (310) comportant des surfaces (312), un hydrogel (316), des cellules (314), dans laquelle les cellules (314) sont disposées dans des monocouches sur au moins 50 % d'une superficie totale des surfaces (312) de l'échafaudage (310), et les cellules (314) sont fixées aux surfaces (312) de l'échafaudage (310), et l'hydrogel (316) est fixé aux cellules (314), **caractérisée en ce que** l'échafaudage (310), les cellules (314) et l'hydrogel (316) sont disposées de sorte que les cellules (314) soient prises en sandwich dans des espaces existant entre l'hydrogel (316) et les surfaces (312) de l'échafaudage (310), permettant ainsi une mobilité et une prolifération des cellules (314) dans les espaces existant entre l'hydrogel (316) et les surfaces (312) de l'échafaudage (310), et empêchant la mobilité et la prolifération des cellules (314) à des positions extérieures aux espaces existant entre l'hydrogel (316) et les surfaces (312) de l'échafaudage (310).

2. Structure d'encapsulation pouvant être implantée (300) selon la revendication 1, dans laquelle les cellules (314) sont disposées dans les monocouches sur au moins 70 % de la superficie totale des surfaces (312) de l'échafaudage (310).

3. Structure d'encapsulation pouvant être implantée (300) selon la revendication 1, dans laquelle les cellules (314) sont disposées dans les monocouches sur au moins 90 % de la superficie totale des surfaces (312) de l'échafaudage (310).

4. Structure d'encapsulation pouvant être implantée (300) selon la revendication 1, comprenant en outre :
une chambre (57) dans laquelle sont contenus l'échafaudage (310), les cellules (314) et l'hydrogel (316).

5. Structure d'encapsulation pouvant être implantée (300) selon la revendication 4, comprenant en outre :
une membrane externe (58) qui entoure au moins une partie de la chambre (57).

6. Structure d'encapsulation pouvant être implantée (300) selon la revendication 1, dans laquelle les cellules (314) sont des cellules différenciées, qui sont fixées aux surfaces (312) de l'échafaudage (310).

7. Structure d'encapsulation pouvant être implantée (300) selon la revendication 6, dans laquelle les cellules différenciées sont des cellules à terminaisons différenciées, qui sont fixées aux surfaces (312) de l'échafaudage (310).

8. Structure d'encapsulation pouvant être implantée (300) selon la revendication 1, dans laquelle les cellules (314) sont des cellules souches, qui sont fixées aux surfaces (312) de l'échafaudage (310).

9. Structure d'encapsulation pouvant être implantée (300) selon la revendication 1, dans laquelle les cellules (314) sont génétiquement modifiées pour produire une protéine capteur fluorescente comportant un site de liaison avec un analyte, la protéine capteur fluorescente étant conçue pour émettre une lumière fluorescente en réponse à une lumière d'excitation, l'analyte étant éventuellement du glucose.

10. Structure d'encapsulation pouvant être implantée (300) selon l'une quelconque des revendications 1 à 9, dans laquelle l'échafaudage (310) comprend des microsphères, remplissant éventuellement des membranes fibreuses creuses.

11. Structure d'encapsulation pouvant être implantée (300) selon l'une quelconque des revendications 1 à 9, dans laquelle l'échafaudage (310) comprend des fibres, éventuellement sous forme de tresse ou sous forme chaotique ou aléatoire.

12. Structure d'encapsulation pouvant être implantée (300) selon l'une quelconque des revendications 1 à 9, dans laquelle l'échafaudage (310) comprend une structure spongieuse comportant une pluralité de ports internes interconnectés, ayant un diamètre éventuellement supérieur à 50 µm, éventuellement un diamètre supérieur à 100 µm.

13. Structure d'encapsulation pouvant être implantée (300) selon l'une quelconque des revendications 1 à 9, dans laquelle l'échafaudage (310) est une structure rigide (28).

14. Structure d'encapsulation pouvant être implantée (300) selon la revendication 13, dans laquelle la structure rigide (28) est formée de façon à définir une pluralité de puits (30).

15. Procédé de fabrication d'une structure d'encapsulation pouvant être implantée (300) selon la revendication 1, contenant des cellules, destinée à une implantation chez un sujet humain, comprenant : l'utilisation d'un échafaudage tridimensionnel sensiblement non dégradable (310) comportant des surfaces (312) de fixation et de croissance de cellules; l'ensemencement de cellules (314) sur les surfaces (312) de l'échafaudage (310) et le fait de laisser les cellules proliférées pour atteindre une confluence d'au moins 70 %, **caractérisé en ce que**, avant que les cellules (314) ne forment des structures tridimensionnelles sur 50 % d'une superficie totale des surfaces (312) de l'échafaudage (310), le remplissage, avec un hydrogel (316), d'un volume de l'échafaudage (310) qui n'est pas encore occupé par les cellules (314), empêchant ainsi une prolifération supplémentaire des cellules (314), ce par quoi les cellules (314) sont disposées en monocouches sur au moins 50 % d'une superficie totale des surfaces (312) de l'échafaudage (310).
